(19) Europäisches Patentamt
European Patent Office
Office européen des brevets

(11) **EP 3 988 175 A1**

(12) **EUROPEAN PATENT APPLICATION**
published in accordance with Art. 153(4) EPC


(43) Date of publication:
**27.04.2022 Bulletin 2022/17**

(21) Application number: **20827927.3**

(22) Date of filing: **19.06.2020**

(51) International Patent Classification (IPC):
***A61P 35/00*** *(2006.01)* ***A61P 43/00*** *(2006.01)*
***A61K 31/519*** *(2006.01)* ***A61K 31/5365*** *(2006.01)*

(52) Cooperative Patent Classification (CPC):
**A61K 31/519; A61K 31/5365; A61P 35/00; A61P 43/00**

(86) International application number:
**PCT/JP2020/024113**

(87) International publication number:
**WO 2020/256096 (24.12.2020 Gazette 2020/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.06.2019 JP 2019115496**



(71) Applicant: **Taiho Pharmaceutical Co., Ltd.**
**Chiyoda-ku**
**Tokyo 101-8444 (JP)**

(72) Inventors:
- **KOBA, Kazuo**
**Tokyo 101-8444 (JP)**
- **SHIMOMURA, Toshiyasu**
**Tsukuba-shi, Ibaraki 300-2611 (JP)**

(74) Representative: **Hoffmann Eitle**
**Patent- und Rechtsanwälte PartmbB**
**Arabellastraße 30**
**81925 München (DE)**


(54) **METHOD FOR TREATING MALIGNANT TUMOR**


(57) A method for treating a malignant tumor of a human patient, the method comprising administering trans-3-amino-1-methyl-3-(4-(3-phenyl-5H-imidazo[1,2-c]pyrido[3,4-e] [1,3]oxazin-2-yl)phenyl)cyclobutanol (Compound 1) or a salt thereof to a patient in need of the treatment according to an administration schedule in which the Compound 1 or a salt thereof is administered in a dose of 4 mg/body/day to 160 mg/body/day in terms of Compound 1 for 4 consecutive days, followed by a prescribed withdrawal period of 3 days.


Fig. 4

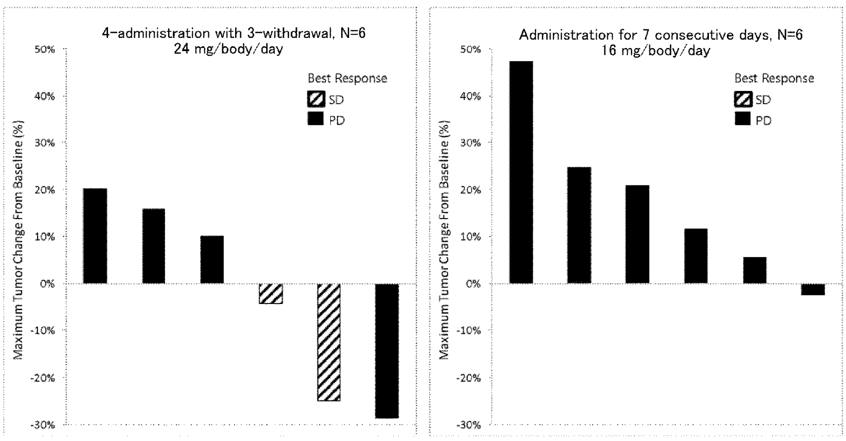


EP 3 988 175 A1

## Description

Technical Field

Cross-Reference to Related Applications

This application claims priority based on Japanese Patent Application No. 2019-115496 filed on June 21, 2019, the entire contents of which are incorporated herein by reference.

The present invention relates to a method for treating a malignant tumor with an imidazooxazine compound having AKT inhibitory activity and antitumor activity.

Background Art

AKT is a serine/threonine kinase acting downstream of phosphatidylinositol 3-kinase (PI3 kinase), which is activated by a receptor tyrosine kinase signal. As for the function thereof, AKT has been reported to play an important role in tumorigenesis such as cell proliferation, apoptosis resistance, angiogenesis, metastasis and invasion, as well as glucose metabolism and lipid metabolism (Non-patent Literature (NPL) 1). AKT is frequently activated or highly expressed in many cancers (renal cell cancer, gastric cancer, breast cancer, lung cancer, colorectal cancer, pancreatic cancer, ovarian cancer, hepatocellular carcinoma, multiple myeloma, lymphoma, leukemia, head and neck cancer, melanoma, and the like), and genetic amplification or activating mutation has been reported in some cancers (NPL 2).

Trans-3-amino-1-methyl-3-(4-(3-phenyl-5H-imidazo[1,2-c]pyrido[3,4-e][1,3]oxazin-2-yl)phenyl)cyclobutanol (hereinafter also referred to as "Compound 1") or a salt thereof, which is an imidazooxazine compound, is known as a highly potent and highly selective oral allosteric AKT inhibitor (Patent Literature (PTL) 1) .

Although many AKT inhibitors have been developed for use as medicaments, no compounds have been approved as medicaments at present. This is because grade 3 or higher side effects such as rash, itching, hyperglycemia, and stomatitis have been observed in clinical trials, and diarrhea, nausea, vomiting, fatigue, loss of appetite, and skin dryness have occurred as other major side effects. There are also potential risks of gastrointestinal disorders, metabolism disorders, and skin disorders (NPL 3 and NPL 4).

As for the study of administration schedules to reduce the side effects of AKT inhibitors, an administration method for AZD5363 including a prescribed withdrawal period has been reported. In this report, the recommend doses (RD) were determined for the following schedules: a schedule in which one course consists of 7 days, and in which AZD5363 is administered twice a day for 7 consecutive days, i.e., a total of 14 times; a schedule in which one course consists of 7 days, and in which AZD5363 is administered twice a day for 2 consecutive days, i.e., a total of 4 times, followed by a prescribed withdrawal period of 5 days; and a schedule in which one course consists of 7 days, and in which AZD5363 is administered twice a day for 4 consecutive days, i.e., a total of 8 times, followed by a prescribed withdrawal period of 3 days (NPL 5).

The half-life of AZD5363, which acts in a competitive inhibition manner, in humans is about 6 hours. The half-life of allosteric Compound 1, which acts in a different inhibition manner, in mice is about 2.3 hours; however, the pharmacokinetics thereof in humans have not been clarified (NPL 6). In investigating the optimal administration schedule of Compound 1 in humans, the nonclinical properties are not always applicable in humans; and even if the target enzymes are the same, the pharmacokinetic properties may differ significantly. Thus, the pharmacokinetic properties of even AKT inhibitors such as AZD5363 cannot always be used without modification as a reference.

Therefore, a method for treating a malignant tumor using an AKT inhibitor capable of suppressing the occurrence of side effects while exhibiting excellent antitumor effects is highly desired.

It is known that Compound 1 or a salt thereof is administered for 7 consecutive days in combination with Compound 2 or a salt thereof (PTL 3). However, a combination of administration of Compound 1 or a salt thereof for 4 days followed by prescribed withdrawal for 3 days and administration of Compound 2 or a salt thereof for 21 consecutive days is not disclosed.

Citation List

Patent Literature

PTL 1: WO2012/137870
PTL 2: WO2013/108809
PTL 3: US Patent Application Publication No. 20190350932

Non-patent Literature

NPL 1: Cell 2007, 129, p1261-1274
NPL 2: Ann Oncol 2010, 21, p683-691
NPL 3: J Clin Oncol. 2011, 29:4688-4695.
NPL 4: Cancer Res. 2013, 73 (Suppl 8): Abstract LB-197
NPL 5: Clin Cancer Res 2018, May 1, 24(9): 2050-2059, (doi: 10.1158/1078-0432.CCR-17-2260)
NPL 6: Poster presentation material for the conference "EORTC-NCI-AACR 2017," "Characterization of TAS-117, a novel, highly potent and selective inhibitor of AKT"

Summary of Invention

Technical Problem

An object of the present invention is to provide a method for treating a malignant tumor with an imidazooxazine compound having AKT inhibitory activity while reducing side effects.

Solution to Problem

The present inventors found that administration of Compound 1 or a salt thereof, which exhibits a highly potent and highly selective AKT inhibitory activity, according to a specific administration method enables highly effective treatment of malignant tumors while reducing side effects.

More specifically, the present invention provides the inventions according to Items 1 to 58 below.

1. A method for treating a malignant tumor of a human patient, the method comprising administering trans-3-amino-1-methyl-3-(4-(3-phenyl-5H-imidazo[1,2-c]pyrido[3,4-e] [1,3]oxazin-2-yl)phenyl)cyclobutanol (Compound 1) or a salt thereof to a patient in need of the treatment according to an administration schedule, the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days.

2. A therapeutic agent for treating a malignant tumor in a human patient, comprising Compound 1 or a salt thereof, wherein the Compound 1 or a salt thereof is for use in administration to a patient in need of the treatment according to an administration schedule,
the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days.

3. A pharmaceutical composition for treating a malignant tumor in a human patient, comprising Compound 1 or a salt thereof,

wherein the Compound 1 or a salt thereof is for use in administration to a patient in need of the treatment according to an administration schedule,
the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days.

4. Use of Compound 1 or a salt thereof in the manufacture of a therapeutic agent for a malignant tumor in a human patient,

wherein the Compound 1 or a salt thereof is for use in administration to a patient in need of the treatment according to an administration schedule,
the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days.

5. Compound 1 or a salt thereof for use in the treatment of a malignant tumor in a human patient,

wherein the Compound 1 or a salt thereof is for use in administration to a patient in need of the treatment according to an administration schedule,
the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days.

6. Use of Compound 1 or a salt thereof for the treatment of a malignant tumor in a human patient,

wherein the Compound 1 or a salt thereof is for use in administration to a patient in need of the treatment according to an administration schedule,
the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days.

7. A kit for treating a malignant tumor in a human patient, comprising Compound 1 or a salt thereof,

wherein the Compound 1 or a salt thereof is for use in administration to a patient in need of the treatment according to an administration schedule,
the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days.

8. The method, the therapeutic agent, the pharmaceutical composition, the use, the compound or a salt thereof, or the kit according to any one of Items 1 to 7, wherein Compound 1 or a salt thereof is administered singly.
9. The method, the therapeutic agent, the pharmaceutical composition, the use, the compound or a salt thereof, or the kit according to any one of Items 1 to 8, wherein Compound 1 or a salt thereof is administered once a day on an administration day.
10. The method, the therapeutic agent, the pharmaceutical composition, the use, the compound or a salt thereof, or the kit according to any one of Items 1 to 9, wherein the administration schedule includes three courses for a total duration of 21 days, with one course taken as 7 days, in which Compound 1 or a salt thereof is administered for 4 consecutive days each from day 1 to day 4, day 8 to day 11, and day 15 to day 18, and prescribed withdrawal is performed from day 5 to day 7, day 12 to day 14, and day 19 to day 21.
11. The method, the therapeutic agent, the pharmaceutical composition, the use, the compound or a salt thereof, or the kit according to any one of Items 1 to 10, wherein the dose of the Compound 1 or a salt thereof is 4 mg/body/day to 44 mg/body/day.
12. The method, the therapeutic agent, the pharmaceutical composition, the use, the compound or a salt thereof, or the kit according to any one of Items 1 to 11, wherein the dose of the Compound 1 or a salt thereof is 16 mg/body/day to 32 mg/body/day.
13. The method, the therapeutic agent, the pharmaceutical composition, the use, the compound or a salt thereof, or the kit according to any one of Items 1 to 12, wherein the dose of the Compound 1 or a salt thereof is 24 mg/body/day.
14. The method, the therapeutic agent, the pharmaceutical composition, the use, the compound or a salt thereof, or the kit according to any one of Items 1 to 13, wherein the malignant tumor is lung cancer, breast cancer, renal cancer, sarcoma, hematopoietic tumor, central nervous system tumor, liver cancer, ovarian cancer, gastric cancer, rhabdomyosarcoma, endometrial cancer, urinary tract cancer, prostate cancer, colorectal cancer, pancreatic cancer, vulvar cancer, thyroid cancer, skin cancer, or biliary tract cancer.
15. The method, the therapeutic agent, the pharmaceutical composition, the use, the compound or a salt thereof, or the kit according to any one of Items 1 to 14, wherein the malignant tumor is lung cancer, endometrial cancer, urinary tract cancer, breast cancer, prostate cancer, colorectal cancer, biliary tract cancer, ovarian cancer, or multiple myeloma.
16. The method, the therapeutic agent, the pharmaceutical composition, the use, the compound or a salt thereof, or the kit according to any one of Items 1 to 15, wherein the malignant tumor is lung cancer, breast cancer, bladder cancer, colon cancer, rectal cancer, gallbladder cancer, or anal cancer.
17. The method, the therapeutic agent, the pharmaceutical composition, the use, the compound or a salt thereof, or the kit according to any one of Items 1 to 16, wherein a side effect caused by Compound 1 or a salt thereof is reduced.
18. The method, the therapeutic agent, the pharmaceutical composition, the use, the compound or a salt thereof, or the kit according to Item 17, wherein the reduced side effect is maculopapular rash.
19. The method, the therapeutic agent, the pharmaceutical composition, the use, the compound or a salt thereof, or the kit according to Item 17, wherein the reduced side effect is hyperglycemia.

20. The method according to Item 1, further comprising administering (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one (Compound 2) or a salt thereof according to the administration schedule,
the administration schedule including administration of Compound 2 or a salt thereof in combination for 7 consecutive days.
21. The method according to Item 20, wherein Compound 2 or a salt thereof is administered in combination in a dose of 12 mg/body/day to 20 mg/body/day in terms of Compound 2 for 7 consecutive days.
22. The therapeutic agent according to Item 2, which is for a combined use of Compound 1 or a salt thereof with Compound 2 or a salt thereof,

wherein Compound 2 or a salt thereof is administered according to the administration schedule,
the administration schedule including administration of Compound 2 or a salt thereof in combination for 7 consecutive days.

23. The therapeutic agent according to Item 22, wherein Compound 2 or a salt thereof is administered in combination in a dose of 12 mg/body/day to 20 mg/body/day in terms of Compound 2 for 7 consecutive days.
24. The therapeutic agent according to Item 22 or 23, comprising Compound 2 or a salt thereof.
25. The pharmaceutical composition according to Item 3, which is for a combined use of Compound 1 or a salt thereof with Compound 2 or a salt thereof,

wherein Compound 2 or a salt thereof is administered according to the administration schedule,
the administration schedule including administration of Compound 2 or a salt thereof in combination for 7 consecutive days.

26. The pharmaceutical composition according to Item 25, wherein Compound 2 or a salt thereof is administered in combination in a dose of 12 mg/body/day to 20 mg/body/day in terms of Compound 2 for 7 consecutive days.
27. The pharmaceutical composition according to Item 25 or 26, comprising Compound 2 or a salt thereof.
28. The use according to Item 4, wherein the therapeutic agent is for a combined use of Compound 1 or a salt thereof with Compound 2 or a salt thereof, and

Compound 2 or a salt thereof is administered according to the administration schedule,
the administration schedule including administration of Compound 2 or a salt thereof in combination for 7 consecutive days.

29. The use according to Item 28, wherein Compound 2 or a salt thereof is administered in combination in a dose of 12 mg/body/day to 20 mg/body/day in terms of Compound 2 for 7 consecutive days.
30. The compound or a salt thereof according to Item 5, wherein the Compound 1 or a salt thereof is for use in combination with Compound 2 or a salt thereof, and Compound 2 or a salt thereof is administered according to the administration schedule,
the administration schedule including administration of Compound 2 or a salt thereof in combination for 7 consecutive days.
31. The compound or a salt thereof according to Item 30, wherein Compound 2 or a salt thereof is administered in combination in a dose of 12 mg/body/day to 20 mg/body/day in terms of Compound 2 for 7 consecutive days.
32. The use according to Item 6, wherein the Compound 1 or a salt thereof is for use in combination with Compound 2 or a salt thereof, and Compound 2 or a salt thereof is administered according to the administration schedule,
the administration schedule including administration of Compound 2 or a salt thereof in combination for 7 consecutive days.
33. The use according to Item 32, wherein the administration schedule includes administration of Compound 2 or a salt thereof in combination in a dose of 12 mg/body/day to 20 mg/body/day in terms of Compound 2 for 7 consecutive days.
34. The kit according to Item 7, which is for a combined use of Compound 1 or a salt thereof with Compound 2 or a salt thereof, wherein

Compound 2 or a salt thereof is administered according to the administration schedule,
the administration schedule including administration of Compound 2 or a salt thereof in combination for 7 consecutive days.

35. The kit according to Item 34, wherein Compound 2 or a salt thereof is administered in combination in a dose of

12 mg/body/day to 20 mg/body/day in terms of Compound 2 for 7 consecutive days.

36. The kit according to Item 34 or 35, comprising Compound 2 or a salt thereof.

37. A combination of Compound 1 or a salt thereof and Compound 2 or a salt thereof for use in the treatment of a malignant tumor in a human patient,

wherein the Compound 1 or a salt thereof and the Compound 1 or a salt thereof are for use in administration to a patient in need of the treatment according to an administration schedule,

the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days, and in which Compound 2 or a salt thereof is administered in combination for 7 consecutive days.

38. The combination according to Item 37, wherein Compound 2 or a salt thereof is administered in combination in a dose of 12 mg/body/day to 20 mg/body/day in terms of Compound 2 for 7 consecutive days.

39. Use of Compound 1 or a salt thereof and Compound 2 or a salt thereof in the manufacture of a therapeutic agent for a malignant tumor in a human patient,

wherein the Compound 1 or a salt thereof and the Compound 1 or a salt thereof are for use in administration to a patient in need of the treatment according to an administration schedule, the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days, and in which Compound 2 or a salt thereof is administered in combination for 7 consecutive days.

40. The use according to Item 39, wherein Compound 2 or a salt thereof is administered in combination in a dose of 12 mg/body/day to 20 mg/body/day in terms of Compound 2 for 7 consecutive days.

41. Use of Compound 1 or a salt thereof and Compound 2 or a salt thereof for the treatment of a malignant tumor in a human patient,

wherein the Compound 1 or a salt thereof and the Compound 1 or a salt thereof are for use in administration to a patient in need of the treatment according to an administration schedule, the administration schedule including one course for a duration of 7 days in which the Compound 1 or a salt thereof is administered in a dose of 4 mg/body/day to 160 mg/body/day in terms of Compound 1 for 4 consecutive days, followed by a prescribed withdrawal period of 3 days, and in which Compound 2 or a salt thereof is administered in combination for 7 consecutive days.

42. The use according to Item 41, wherein Compound 2 or a salt thereof is administered in combination in a dose of 12 mg/body/day to 20 mg/body/day in terms of Compound 2 for 7 consecutive days.

43. The method, the therapeutic agent, the pharmaceutical composition, the use, the compound or a salt thereof, the combination, or the kit according to any one of Items 20 to 42, wherein Compound 1 or a salt thereof is administered once a day on an administration day.

44. The method, the therapeutic agent, the pharmaceutical composition, the use, the compound or a salt thereof, the combination, or the kit according to any one of Items 20 to 43, wherein the administration schedule includes three courses for a total duration of 21 days, with one course taken as 7 days, in which Compound 1 or a salt thereof is administered for 4 consecutive days each from day 1 to day 4, day 8 to day 11, and day 15 to day 18, and prescribed withdrawal is performed from day 5 to day 7, day 12 to day 14, and day 19 to day 21.

45. The method, the therapeutic agent, the pharmaceutical composition, the use, the compound or a salt thereof, the combination, or the kit according to any one of Items 20 to 44, wherein the dose of the Compound 1 or a salt thereof is 4 mg/body/day to 44 mg/body/day.

46. The method, the therapeutic agent, the pharmaceutical composition, the use, the compound or a salt thereof, the combination, or the kit according to any one of Items 20 to 45, wherein the dose of the Compound 1 or a salt thereof is 16 mg/body/day to 32 mg/body/day.

47. The method, the therapeutic agent, the pharmaceutical composition, the use, the compound or a salt thereof, the combination, or the kit according to any one of Items 20 to 46, wherein the dose of the Compound 1 or a salt thereof is 24 mg/body/day.

48. The method, the therapeutic agent, the pharmaceutical composition, the use, the compound or a salt thereof, the combination, or the kit according to any one of Items 20 to 47, wherein the malignant tumor is a solid cancer.

49. The method, the therapeutic agent, the pharmaceutical composition, the use, the compound or a salt thereof, the combination, or the kit according to any one of Items 20 to 48, wherein the malignant tumor is lung cancer, breast cancer, renal cancer, sarcoma, central nervous system tumor, liver cancer, ovarian cancer, gastric cancer, rhabdomyosarcoma, endometrial cancer, urinary tract cancer, prostate cancer, colorectal cancer, pancreatic cancer, vulvar cancer, thyroid cancer, skin cancer, or biliary tract cancer.

50. The method, the therapeutic agent, the pharmaceutical composition, the use, the compound or a salt thereof, the combination, or the kit according to any one of Items 20 to 49, wherein the malignant tumor is lung cancer,

endometrial cancer, urinary tract cancer, breast cancer, prostate cancer, colorectal cancer, biliary tract cancer, or ovarian cancer.

51. The method, the therapeutic agent, the pharmaceutical composition, the use, the compound or a salt thereof, the combination, or the kit according to any one of Items 20 to 50, wherein the malignant tumor is lung cancer, breast cancer, bladder cancer, colon cancer, rectal cancer, gallbladder cancer, or anal cancer.

52. The method, the therapeutic agent, the pharmaceutical composition, the use, the compound or a salt thereof, the combination, or the kit according to any one of Items 20 to 51, wherein a side effect caused by Compound 1 or a salt thereof is reduced.

53. The method, the therapeutic agent, the pharmaceutical composition, the use, the compound or a salt thereof, the combination, or the kit according to Item 52, wherein the reduced side effect is maculopapular rash.

54. The method, the therapeutic agent, the pharmaceutical composition, the use, the compound or a salt thereof, the combination, or the kit according to Item 52, wherein the reduced side effect is hyperglycemia.

55. The method, the therapeutic agent, the pharmaceutical composition, the use, the compound or a salt thereof, the combination, or the kit according to any one of Items 20 to 54, wherein the malignant tumor is a solid cancer that is confirmed to be positive for genetic aberrations in the fibroblast growth factor (FGF), fibroblast growth factor receptor (FGFR), PTEN, PIK3CA, or AKT for which standard treatment options are not available.

56. The method, the therapeutic agent, the pharmaceutical composition, the use, the compound or a salt thereof, the combination, or the kit according to claim 55, wherein the malignant tumor is a solid cancer that is confirmed to be positive for FGFR genetic aberrations.

57. The method, the therapeutic agent, the pharmaceutical composition, the use, the compound or a salt thereof, the combination, or the kit according to claim 55 or 56, wherein the malignant tumor is a non-small cell lung cancer that is confirmed to be positive for FGFR1 gene amplification.

58. The method, the therapeutic agent, the pharmaceutical composition, the use, the compound or a salt thereof, the combination, or the kit according to any one of claims 55 to 57, wherein the malignant tumor is lung squamous cell cancer that is confirmed to be positive for FGFR1 gene amplification.

Advantageous Effects of Invention

The method according to the present invention is capable of treating a malignant tumor while achieving excellent antitumor effects and reducing side effects.

Brief Description of Drawings

Fig. 1 shows the test results of Example 1.
Fig. 2 shows the test results of Example 2.
Fig. 3 shows the pharmacokinetics of Compound 1 in Example 4.
Fig. 4 shows an antitumor effect on each patient in Example 5 as the percentage change from baseline.

Description of Embodiments

The imidazooxazine compound (chemical name: trans-3-amino-1-methyl-3-(4-(3-phenyl-5H-imidazo[1,2-c]pyrido[3,4-e][1,3]oxazin-2-yl)phenyl)cyclobutanol (Compound 1)) used as an active ingredient in the present invention is represented by the following structural formula (I).

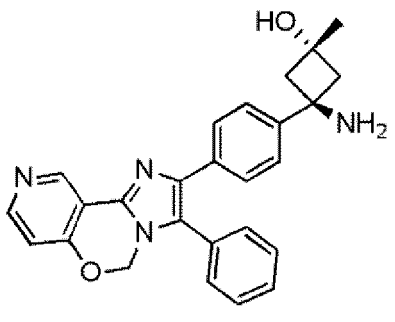

(I)

Compound 1 or a salt thereof is a known compound and can be synthesized, for example, according to the methods disclosed in PTL 1 (WO 2012/137870).

In the present invention, the salts of Compound 1 mean pharmaceutically acceptable salts.

In the present invention, Compound 1 or a salt thereof is administered to a patient in need of treatment of a malignant tumor according to its administration schedule.

In the present invention, the term "administration schedule" refers to a plan that shows the drug's type, amount, duration, procedure, etc., in drug treatment in chronological order, and shows the administration amount, administration method, administration order, administration day, etc. of each drug. The date on which administration is prescribed to be performed and the date on which administration is prescribed to not be performed are determined before the initiation of drug administration. Also, one set of the administration schedule is defined as a "course," and the administration is continued by repeating the course. The administration schedule in which one course that includes drug administration and prescribed withdrawal is repeated is referred to as two courses (an administration schedule in which the one course is repeated twice), three courses (an administration schedule in which the one course is repeated 3 times), and the like.

In the present invention, the term "administration" represents administrating the drug, and "withdrawal" represents not administrating the drug. The term "administration day" refers to a day on which administration is performed, and the term "withdrawal day" refers to a day on which no administration is performed.

The term "prescribed withdrawal" or "prescribed withdrawal period" as used in the present invention indicates not performing drug administration according to a previously prescribed administration schedule, or a period of the same.

In the present invention, the term "non-prescribed withdrawal" or "non-prescribed withdrawal period" indicates not performing drug administration or being unable to perform drug administration due to side effects etc. as a result of the past drug administration although it is a previously prescribed day to perform drug administration, or a period of the same.

With regard to the administration schedule according to the present invention, the term "consecutive" indicates that the administration is performed every day without interruption. The administration for "A consecutive days" means that a compound is administered consecutively for A days.

In the present invention, the expression such as "administration for N days, followed by a prescribed withdrawal period of X days" means that drug administration is performed from day 1 to day (N), and then, prescribed withdrawal is performed from day (N + 1) to day (N + X). This administration method may also be referred to as "N-administration with X-withdrawal." For example, when one course is taken as 7 days, "administration for 4 consecutive days, followed by prescribed withdrawal for 3 days" means that administration is consecutively performed from day 1 to day 4, and prescribed withdrawal is performed from day 5 to day 7; thereafter, administration is resumed from day 8 in the next course. This manner of administration is referred to as "4-administration with 3-withdrawal." For example, when three courses of "administration for 4 consecutive days, followed by prescribed withdrawal for 3 days" are performed for a total duration of 21 days, Compound 1 or a salt thereof is administered for 4 consecutive days each from day 1 to day 4, from day 8 to day 11, and from day 15 to day 18, and prescribed withdrawal is performed from day 5 to day 7, from day 12 to day 14, and from day 19 to day 21.

The term "adverse event" used in the present invention refers to any undesired or unintended disease, or sign of such disease, developed in a patient who has been administered a drug. The causal relationship with the drug is not questioned. The term "side effect" used in the present invention includes any adverse reactions following drug administration regardless of the administration amount (including aberrations in laboratory data). More specifically, the term refers to reactions for which causal relationship between the drug and adverse events can be reasonably established, and in which the causal relationship cannot be denied.

The term "control of side effects" used in the present invention means recovery of developed side effects to grade 1 or less or to the baseline, or means performing appropriate treatment to prevent further exacerbations from occurring even when drug administration is continued. For hyperglycemia, the term means recovery to grade 1 or grade 0.

In the present invention, the term "recommend dose (RD)" refers to an administration amount that can be administered without causing unacceptable side effects, and that is considered to be most preferable in view of safety, efficacy, pharmacokinetics, and pharmacodynamic studies. The recommend dose is determined based on human clinical trials. Specific examples of the recommend dose include administration amounts that have been approved, recommended, or advised by public institutions or organizations, such as the Japan Pharmaceuticals and Medical Devices Agency (PMDA), the U.S. Food and Drug Administration (FDA), and the European Medicines Agency (EMA), and are described in package inserts, interview forms, treatment guidelines, etc. The administration amounts are preferably approved by a public institution of the PMDA, FDA, or EMA.

During analysis of the present invention, Compound 1 or a salt thereof was newly found to remain in the body for an extremely long period of time when administered to humans as demonstrated in Examples described later, which confirmed that the blood half-life was about 49 hours although it was about 2.3 hours when administered to mice. These results indicate that medicaments have specific characteristic differences, as well as different pharmacokinetic characteristics, depending on each compound between in humans and mice. Further, in the present invention, it has been

revealed that Compound 1 or a salt thereof, when administered consecutively for 4 to 7 days, is not sufficiently metabolized, excreted, or the like from the blood, and has accumulation properties. Since it is difficult to predict what pharmacokinetics will be obtained and what and how side effects will occur when such medicaments with a long half-life and accumulation properties are administered consecutively, performing administration for consecutive days should be reluctantly performed.

Nevertheless, in the present invention, the inventors performed administration for 7 consecutive days or administration for 4 days followed by prescribed withdrawal for 3 days by using an administration method that is based on daily administration. The results revealed that the administration based on 4-administration with 3-withdrawal using a specific administration amount can reduce side effects while demonstrating an excellent antitumor effect. In the present invention, a reduction of side effects means a reduction of side effects as compared with the side effects caused by the administration for 7 consecutive days, unless otherwise specified.

Specifically, in the present invention, Compound 1 or a salt thereof can be repeatedly administered in two to four courses. For two courses, the administration is performed according to an administration schedule that includes two courses for a total duration of 14 days, with one course taken as 7 days, in which Compound 1 or a salt thereof is administered for 4 consecutive days each from day 1 to day 4 and day 8 to day 11, and prescribed withdrawal is performed from day 5 to day 7 and day 12 to day 14. For three courses, the administered is performed according to an administration schedule that includes three courses for a total duration of 21 days, with one course taken as 7 days, in which Compound 1 or a salt thereof is administered for 4 consecutive days each from day 1 to day 4, day 8 to day 11, and day 15 to day 18, and prescribed withdrawal is performed from day 5 to day 7, day 12 to day 14, and day 19 to day 21. For four courses, the administered is performed in accordance with an administration schedule that includes four courses for a total duration of 28 days, with one course taken as 7 days, in which Compound 1 or a salt thereof is administered for 4 consecutive days each from day 1 to day 4, day 8 to day 11, day 15 to day 18, and day 22 to 25, and prescribed withdrawal is performed from day 5 to day 7, day 12 to day 14, day 19 to day 21, and day 26 to 28. Repeated administration in three courses is more preferred.

In the present invention, the daily dose for human of Compound 1 or a salt thereof on an administration day is 4 mg/body/day to 160 mg/body/day as the amount of the first administration of Compound 1. The dose is preferably 4 mg/body/day to 44 mg/body/day, more preferably 16 mg/body/day to 32 mg/body/day, still more preferably 16 mg/body/day or 24 mg/body/day, and most preferably 24 mg/body/day. The daily dose may be administered in divided doses; however, it is preferable to administer the dose above once a day. (For example, when administration is performed for 4 consecutive days in 7 days, the dose above is preferably administered 4 times in consecutive days during the 7 days.)

In the present invention, Compound 1 or a salt thereof is administered preferably according to an administration schedule that includes one course for a duration of 7 days in which a dose of 4 mg to 160 mg is administered for 4 consecutive days, followed by a prescribed withdrawal period of 3 days, more preferably according to an administration schedule that includes one course for a duration of 7 days in which a dose of 4 mg to 44 mg is administered for 4 consecutive days, followed by a prescribed withdrawal period of 3 days, still more preferably according to an administration schedule that includes one course for a duration of 7 days in which a dose of 16 mg to 32 mg is administered for 4 consecutive days, followed by a prescribed withdrawal period of 3 days, and even more preferably according to an administration schedule that includes one course for a duration of 7 days in which a dose of 16 mg or 24 mg is administered for 4 consecutive days, followed by a prescribed withdrawal period of 3 days. The administration is particularly preferably performed according to an administration schedule that includes one course for a duration of 7 days in which Compound 1 or a salt thereof is administered in a dose per person per day of 24 mg/body/day on each administration day for 4 consecutive days, followed by a prescribed withdrawal period of 3 days. The administration is most preferably performed in three courses for a total duration of 21 days by repeating, three times, one course that includes administration of Compound 1 or a salt thereof in a dose per person per day of 24 mg/body/day on each administration day for 4 consecutive days, followed by a prescribed withdrawal period of 3 days.

When administration based on 4-administration with 3-withdrawal is performed, Compound 1 or a salt thereof may be administered alone without using other antitumor agents in combination. In the present specification, the administration of Compound 1 or a salt thereof without a combined use of other active ingredients, such as other antitumor agents, is referred to as "single administration." As long as other active ingredients are not used in combination, the term "single administration" also encompasses, for example, embodiments of administering a preparation comprising a combination of Compound 1 or a salt thereof with an excipient or other components. In another embodiment of the present invention, Compound 1 or a salt thereof can also be administered based on 4-administration with 3-withdrawal in combination with other drugs. Examples of such drugs usable in combination include (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one. In the present invention, this compound is simply referred to as "Compound 2." Compound 2 is a disubstituted benzene alkynyl compound having the following structure. Compound 2 is described as Example Compound 2 in PTL 2 (WO 2013/108809). It has been reported that Compound 2 or a salt thereof has excellent FGFR inhibitory activity and inhibits the ability of receptor protein tyrosine kinases (FGFR1, FGFR3, and FGFR4) to phosphorylate tyrosine in the substrate peptide sequence (PTL 2).

Fibroblast growth factors (FGFs) are expressed in various tissues, and are one of the growth factors that regulate cell proliferation and differentiation. The physiological activity of the FGFs is mediated by fibroblast growth factor receptors (FGFRs), which are specific cell surface receptors. FGFRs belong to a receptor protein tyrosine kinase family, and comprise an extracellular ligand-binding domain, a single transmembrane domain, and an intracellular tyrosine kinase domain. Four types of FGFRs (FGFR1, FGFR2, FGFR3, and FGFR4) have been heretofore identified. FGFRs bind to FGFs to form dimers, and are activated by phosphorylation. Activation of the receptors induces mobilization and activation of specific downstream signal transduction molecules, thereby developing physiological functions. Some reports have been made about the relationship between aberrant FGF/FGFR signaling and various human tumors. Aberrant activation of FGF/FGFR signaling in human tumors is considered to be attributable to overexpression of FGFRs and/or gene amplification, gene mutation, chromosomal translocation, insertion, and inversion, gene fusion, or an autocrine or paracrine mechanism by overproduction of FGFs (ligands).

In a preferred embodiment, the present invention can be used for tumors that have aberration in FGFR, PTEN, PIK3CA, or AKT genes. Genetic aberrations include gene amplification, gene mutation, chromosomal translocation, insertion, and inversion, gene fusion, genetic rearrangement, etc. Some of the genetic aberrations in tumors have already been reported in documents available to persons skilled in the art. The genetic aberrations can be detected by methods known to persons skilled in the art, for example, pyrosequence, DNA sequencing including next-generation sequencing (NGS), PCR-based methods including allele-specific PCR chain reactions, microarray-based comparative genomic hybridization (aCGH), fluorescence *in situ* hybridization (FISH), and chromogenic *in situ* hybridization (CISH).

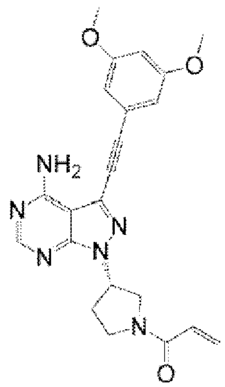

In the present invention, Compound 2 or a salt thereof described above may be synthesized by any method, and may be synthesized, for example, according to the production method described in PTL 2.

In the present invention, the salts of Compound 2 mean pharmaceutically acceptable salts.

In a preferred embodiment of the present invention, Compound 1 or a salt thereof may be used in combination with Compound 2 or a salt thereof.

As used in the present invention, expressions such as "combined use" or "used in combination" are intended to define a therapy that uses a combination of two or more compounds or drugs. Therefore, the use of compounds or drugs "in combination" and a "combined use" of compounds or drugs in the present invention can refer to administering compounds or drugs according to part of the same overall administration schedule. The doses of the two or more compounds or drugs can differ from each other; they can be administered simultaneously or at different times. Thus, compounds or drugs used in combination are understood as being able to be administered consecutively (e.g., one after another) or simultaneously in the same pharmaceutical preparation (i.e., together) or in different pharmaceutical preparations (i.e. separately). When administered simultaneously, the same preparation is administered as a single preparation, and different preparations are administered as non-integrated preparations.

Examples of combinations of the daily dose of Compound 1 or a salt thereof on the administration day with the daily dose of Compound 2 according to the present disclosure include the following:

4 mg/body/day to 160 mg/body/day of Compound 1 of the present disclosure or a salt thereof and 12 mg/body/day to 20 mg/body/day of Compound 2,

4 mg/body/day to 44 mg/body/day of Compound 1 of the present disclosure or a salt thereof and 12 mg/body/day to 20 mg/body/day of Compound 2,

16 mg to 32 mg/body/day of Compound 1 of the present disclosure or a salt thereof and 12 mg/body/day to 20 mg/body/day of Compound 2,

16 mg or 24 mg/body/day of Compound 1 of the present disclosure or a salt thereof and 12 mg/body/day to 20 mg/body/day of Compound 2,

4 mg/body/day of Compound 1 of the present disclosure or a salt thereof and 12 mg/body/day to 20 mg/body/day of Compound 2,
8 mg/body/day of Compound 1 of the present disclosure or a salt thereof and 12 mg/body/day to 20 mg/body/day of Compound 2,
12 mg/body/day of Compound 1 of the present disclosure or a salt thereof and 12 mg/body/day to 20 mg/body/day of Compound 2,
16 mg/body/day of Compound 1 of the present disclosure or a salt thereof and 12 mg/body/day to 20 mg/body/day of Compound 2,
20 mg/body/day of Compound 1 of the present disclosure or a salt thereof and 12 mg/body/day to 20 mg/body/day of Compound 2,
24 mg/body/day of Compound 1 of the present disclosure or a salt thereof and 12 mg/body/day to 20 mg/body/day of Compound 2,
32 mg/body/day of Compound 1 of the present disclosure or a salt thereof and 12 mg/body/day to 20 mg/body/day of Compound 2,
44 mg/body/day of Compound 1 of the present disclosure or a salt thereof and 12 mg/body/day to 20 mg/body/day of Compound 2,
60 mg/body/day of Compound 1 of the present disclosure or a salt thereof and 12 mg/body/day to 20 mg/body/day of Compound 2,
80 mg/body/day of Compound 1 of the present disclosure or a salt thereof and 12 mg/body/day to 20 mg/body/day of Compound 2,
100 mg/body/day of Compound 1 of the present disclosure or a salt thereof and 12 mg/body/day to 20 mg/body/day of Compound 2,
120 mg/body/day of Compound 1 of the present disclosure or a salt thereof and 12 mg/body/day to 20 mg/body/day of Compound 2,
160 mg/body/day of Compound 1 of the present disclosure or a salt thereof and 12 mg/body/day to 20 mg/body/day of Compound 2,
4 mg/body/day of Compound 1 of the present disclosure or a salt thereof and 12 mg/body/day of Compound 2,
8 mg/body/day of Compound 1 of the present disclosure or a salt thereof and 12 mg/body/day of Compound 2,
12 mg/body/day of Compound 1 of the present disclosure or a salt thereof and 12 mg/body/day of Compound 2,
16 mg/body/day of Compound 1 of the present disclosure or a salt thereof and 12 mg/body/day of Compound 2,
20 mg/body/day of Compound 1 of the present disclosure or a salt thereof and 12 mg/body/day of Compound 2,
24 mg/body/day of Compound 1 of the present disclosure or a salt thereof and 12 mg/body/day of Compound 2,
32 mg/body/day of Compound 1 of the present disclosure or a salt thereof and 12 mg/body/day of Compound 2,
44 mg/body/day of Compound 1 of the present disclosure or a salt thereof and 12 mg/body/day of Compound 2,
60 mg/body/day of Compound 1 of the present disclosure or a salt thereof and 12 mg/body/day of Compound 2,
80 mg/body/day of Compound 1 of the present disclosure or a salt thereof and 12 mg/body/day of Compound 2,
100 mg/body/day of Compound 1 of the present disclosure or a salt thereof and 12 mg/body/day of Compound 2,
120 mg/body/day of Compound 1 of the present disclosure or a salt thereof and 12 mg/body/day of Compound 2,
160 mg/body/day of Compound 1 of the present disclosure or a salt thereof and 12 mg/body/day of Compound 2,
4 mg/body/day of Compound 1 of the present disclosure or a salt thereof and 16 mg/body/day of Compound 2,
8 mg/body/day of Compound 1 of the present disclosure or a salt thereof and 16 mg/body/day of Compound 2,
12 mg/body/day of Compound 1 of the present disclosure or a salt thereof and 16 mg/body/day of Compound 2,
16 mg/body/day of Compound 1 of the present disclosure or a salt thereof and 16 mg/body/day of Compound 2,
20 mg/body/day of Compound 1 of the present disclosure or a salt thereof and 16 mg/body/day of Compound 2,
24 mg/body/day of Compound 1 of the present disclosure or a salt thereof and 16 mg/body/day of Compound 2,
32 mg/body/day of Compound 1 of the present disclosure or a salt thereof and 16 mg/body/day of Compound 2,
44 mg/body/day of Compound 1 of the present disclosure or a salt thereof and 16 mg/body/day of Compound 2,
60 mg/body/day of Compound 1 of the present disclosure or a salt thereof and 16 mg/body/day of Compound 2,
80 mg/body/day of Compound 1 of the present disclosure or a salt thereof and 16 mg/body/day of Compound 2,
100 mg/body/day of Compound 1 of the present disclosure or a salt thereof and 16 mg/body/day of Compound 2,
120 mg/body/day of Compound 1 of the present disclosure or a salt thereof and 16 mg/body/day of Compound 2,
160 mg/body/day of Compound 1 of the present disclosure or a salt thereof and 16 mg/body/day of Compound 2,
4 mg/body/day of Compound 1 of the present disclosure or a salt thereof and 20 mg/body/day of Compound 2,
8 mg/body/day of Compound 1 of the present disclosure or a salt thereof and 20 mg/body/day of Compound 2,
12 mg/body/day of Compound 1 of the present disclosure or a salt thereof and 20 mg/body/day of Compound 2,
16 mg/body/day of Compound 1 of the present disclosure or a salt thereof and 20 mg/body/day of Compound 2,
20 mg/body/day of Compound 1 of the present disclosure or a salt thereof and 20 mg/body/day of Compound 2,
24 mg/body/day of Compound 1 of the present disclosure or a salt thereof and 20 mg/body/day of Compound 2,

32 mg/body/day of Compound 1 of the present disclosure or a salt thereof and 20 mg/body/day of Compound 2, 44 mg/body/day of Compound 1 of the present disclosure or a salt thereof and 20 mg/body/day of Compound 2, 60 mg/body/day of Compound 1 of the present disclosure or a salt thereof and 20 mg/body/day of Compound 2, 80 mg/body/day of Compound 1 of the present disclosure or a salt thereof and 20 mg/body/day of Compound 2, 100 mg/body/day of Compound 1 of the present disclosure or a salt thereof and 20 mg/body/day of Compound 2, 120 mg/body/day of Compound 1 of the present disclosure or a salt thereof and 20 mg/body/day of Compound 2, and 160 mg/body/day of Compound 1 of the present disclosure or a salt thereof and 20 mg/body/day of Compound 2.

In this embodiment, Compound 2 or a salt thereof can be administered in parallel according to the following administration schedules while Compound 1 or a salt thereof is administered according to the administration schedules described above. The administration schedule is not particularly limited. For example, Compound 2 or a salt thereof is administered in combination in a dose of 12 mg/body/day to 20 mg/body/day, preferably 20 mg/body/day in terms of Compound 2 for 7 consecutive days.

In a typical embodiment, Compound 1 or a salt thereof is administered according to an administration schedule that includes three courses for a total duration of 21 days, with one course taken as 7 days, in which Compound 1 or a salt thereof is administered once a day in a dose of 4 mg/body/day to 160 mg/body/day (preferably 4 mg/body/day to 44 mg/body/day, more preferably 16 mg/body/day to 32 mg/body/day, still more preferably 16 mg/body/day or 24 mg/body/day, and even more preferably 24 mg/body/day) in terms of Compound 1 for 4 consecutive days each from day 1 to day 4, day 8 to day 11, and day 15 to day 18, and prescribed withdrawal is performed from day 5 to day 7, day 12 to day 14, and day 19 to day 21. In parallel with this, for example, Compound 2 or a salt thereof is administered in combination in a dose of 12 mg/body/day to 20 mg/body/day, preferably 20 mg/body/day in terms of Compound 2 for 21 consecutive days.

The administration schedule for the antitumor agent of the present disclosure can be appropriately selected according to the cancer type, disease stage, and the like.

In another embodiment, products comprising Compound 1 or a salt thereof and Compound 2 or a salt thereof (e.g., therapeutic agents, pharmaceutical compositions, and kits) are provided as combined preparations that are used simultaneously, sequentially, or at an interval.

The active ingredients of Compound 1 or a salt thereof and Compound 2 or a salt thereof of the present invention may each be formulated into multiple dosage forms or may be collectively formulated into a single dosage form according to the administration form and/or administration schedule of each active ingredient. Further, preparations may be produced and sold in a single package suitable for combined administration, or preparations may be produced and sold in separate packages. Therefore, the therapeutic agent, the pharmaceutical composition, the kit, etc., of the present invention "comprising Compound 1 or a salt thereof and Compound 2 or a salt thereof" encompass not only those obtained by formulating these compounds or a salt thereof into a single dosage form but also those obtained by formulating them into multiple dosage forms.

In the present invention, "cancer" or "tumor" refers to physiological conditions of a mammal characterized in that normal control is not accepted due to gene mutation in cells, resulting in unregulated cell growth. "Cancer" and "tumor" have the same meaning in the present invention and are used interchangeably.

Examples of tumors targeted by the present invention include, but are not particularly limited to, epithelial cancers (e.g., respiratory cancer, digestive organ cancer, urogenital cancer, secretory system cancer, skin cancer, and squamous cell cancer), mesothelioma, breast cancer, sarcoma, hematopoietic tumor, central nervous system tumor, and peripheral nervous system tumor. Solid cancers are malignant tumors that develop in any organ and tissue, except for hematopoietic tumors. Specific examples include epithelial cancer, mesothelioma, breast cancer, sarcoma, central nervous system tumor, and peripheral nervous system tumor.

Specific examples of respiratory cancer include lung cancer (e.g., non-small cell lung cancer (e.g., adenocarcinoma, large cell cancer, and lung squamous cell cancer), and small cell lung cancer). Specific examples of digestive organ cancer include esophageal cancer, gastric cancer, duodenal cancer, liver cancer, biliary tract cancer (e.g., gallbladder cancer, and bile duct cancer), pancreatic cancer, and colorectal cancer (e.g., colon cancer, rectal cancer, and anal cancer). Specific examples of urogenital cancer include ovarian cancer, uterine cancer (e.g., cervical cancer, uterine body cancer, and endometrial cancer), urinary tract cancer (e.g., renal pelvis cancer, bladder cancer, and urethra cancer), prostate cancer, renal cancer (renal cell cancer), testicular tumor, and vulvar cancer. Specific examples of secretory system cancer include neuroendocrine tumor and thyroid cancer. Specific examples of mesothelioma include pleural mesothelioma, peritoneal mesothelioma, pericardial mesothelioma, and testicular mesothelioma. Specific examples of sarcoma include bone and soft tissue tumor, and rhabdomyosarcoma. Specific examples of hematopoietic tumor include leukemia, malignant lymphoma, and multiple myeloma. Specific examples of central nervous system tumor include brain tumor. Specific examples of peripheral nervous system tumor include malignant schwannoma.

Of these, epithelial cancer, breast cancer, sarcoma, hematopoietic tumor, central and nervous system tumor are preferred; respiratory cancer, digestive organ cancer, urogenital cancer, secretory system cancer, skin cancer, breast

cancer, sarcoma, hematopoietic tumor, and central nervous system tumor are more preferred; lung cancer, breast cancer, renal cancer, sarcoma, hematopoietic tumor, central nervous system tumor, liver cancer, ovarian cancer, gastric cancer, rhabdomyosarcoma, endometrial cancer, urinary tract cancer, prostate cancer, colorectal cancer, pancreatic cancer, vulvar cancer, thyroid cancer, skin cancer, biliary tract cancer, and the like are still more preferred; lung cancer, endometrial cancer, urinary tract cancer, breast cancer, prostate cancer, colorectal cancer, biliary tract cancer, ovarian cancer, multiple myeloma, and the like are even more preferred; and lung cancer, breast cancer, bladder cancer, colon cancer, rectal cancer, gallbladder cancer, anal cancer, and the like are furthermore preferred. Lung cancer is still even more preferred. Non-small cell lung cancer is particularly preferred. Lung squamous cell cancer is most preferred.

In the present invention, the tumor applied when Compound 1 or a salt thereof is used in combination with Compound 2 or a salt thereof is a solid cancer, preferably a solid cancer with a genetic mutation, and more preferably a solid cancer that is confirmed to be positive for genetic aberrations in the fibroblast growth factor (FGF), fibroblast growth factor receptor (FGFR), PTEN, PIK3CA, or AKT.

Specific examples of solid cancers include those mentioned above, such as epithelial cancer (e.g., respiratory cancer, digestive organ cancer, urogenital cancer, secretory system cancer, skin cancer, and squamous cell cancer), breast cancer, sarcoma, and central nervous system tumor. Specific examples of respiratory cancer include lung cancer. Specific examples of digestive organ cancer include esophageal cancer, gastric cancer, duodenal cancer, liver cancer, biliary tract cancer, pancreatic cancer, and colorectal cancer. Specific examples of urogenital cancer include ovarian cancer, uterine cancer, urinary tract cancer, prostate cancer, renal cancer, and vulvar cancer. Specific examples of secretory system cancer include neuroendocrine tumor and thyroid cancer. Specific examples of sarcoma include bone and soft tissue tumor, and rhabdomyosarcoma. Specific examples of central nervous system tumor include brain tumor.

Of these, the solid cancer is preferably epithelial cancer, breast cancer, sarcoma, or central nervous system tumor, more preferably respiratory cancer, digestive organ cancer, urogenital cancer, secretory system cancer, skin cancer, breast cancer, sarcoma, or central nervous system tumor, still more preferably lung cancer, breast cancer, renal cancer, sarcoma, central nervous system tumor, liver cancer, ovarian cancer, gastric cancer, rhabdomyosarcoma, endometrial cancer, urinary tract cancer, prostate cancer, colorectal cancer, pancreatic cancer, vulvar cancer, thyroid cancer, skin cancer, biliary tract cancer, or the like, even more preferably lung cancer, endometrial cancer, urinary tract cancer, breast cancer, prostate cancer, colorectal cancer, biliary tract cancer, ovarian cancer, or the like, and furthermore preferably lung cancer, breast cancer, bladder cancer, colon cancer, rectal cancer, gallbladder cancer, anal cancer, or the like. Lung cancer is still even more preferred. Non-small cell lung cancer is particularly preferred. Lung squamous cell cancer is most preferred.

The "treatment" of a malignant tumor according to the present invention includes postoperative adjuvant chemotherapy performed to prevent recurrence after surgical removal of the tumor, and preoperative adjuvant chemotherapy performed in advance for surgically removing the tumor.

The treatment in the present invention includes procedures performed for the purpose of curing or ameliorating diseases, or for the purpose of suppressing disease progression or relapse, or alleviating symptoms. The treatment includes administration of drugs before or after surgical procedure, or administration of drugs during, before, or after radiation therapy.

When used as a medicament, Compound 1 or a salt thereof may be in any administration form depending on the therapeutic purpose. Examples of the form include oral preparations (e.g., tablets, coated tablets, powders, granules, capsules, and solutions), injections, suppositories, patches, and ointments. Compound 1 or a salt thereof is preferably in the form of an oral preparation. These preparations can be obtained by performing formulation according to a conventional formulation method commonly known in the art using a pharmaceutically acceptable carrier or the like.

Examples of pharmaceutically acceptable carriers include those that are widely used in typical drugs, such as excipients, binders, disintegrators, lubricants, diluents, solubilizing agents, suspending agents, tonicity agents, pH adjusters, buffers, stabilizers, colorants, flavoring agents, and odor-masking agents.

The preparation of the present invention comprising Compound 1 or a salt thereof may be provided as a kit preparation containing an instruction manual that states that Compound 1 or a salt thereof is administered according to an administration schedule in which Compound 1 or a salt thereof is administered in a dose of 4 mg/body/day to 160 mg/body/day for 4 consecutive days, followed by a prescribed withdrawal period of 3 days. Specific examples of the "instruction manual" include package inserts and brochures. The kit preparation containing an instruction manual may be one in which the instruction manual is printed on or attached to the package of the kit preparation, or may be one in which the instruction manual is enclosed together with Compound 1 or a salt thereof in the package of the kit preparation.

The severity of adverse events in the present invention can be evaluated according to the Common Terminology Criteria for Adverse Events (CTCAE), Version 4.03 or 5.0, of the National Cancer Institute (NCI).

In the present invention, the antitumor effect can be determined according to the RECIST guidelines, version 1.1 (European Journal of Cancer 45 (2009), 228-247) and by comprehensive evaluation of tumor shrinkage between target lesions (lesions having a size equal to or larger than the size that can be measured according to the slice thickness by a diagnostic method (e.g., MRI, CT, or X-ray) used at the time of registration and non-target lesions (all lesions other

than the target lesions).

The antitumor agent according to the present disclosure can be used for the treatment of cancer. The "antitumor effect" on cancer patients subjected to treatment according to the treatment regimens, e.g., the combination therapy described in the present specification, is evaluated based on, for example, at least one of the following: PFS (progression-free survival), DCR (disease control rate), DOR (duration of response), OS (overall survival), ORR (objective response rate), DCR (disease control rate), TTR (time to response), and PROs (patient-reported outcomes). In one embodiment, when the target is a solid cancer, the effect of the combination therapy described in the present specification on tumors is evaluated according to the RECIST 1.1 standard (criteria for effect on solid cancer), and the antitumor effect is represented by SD (stable disease), PR (partial response), CR (complete response), and PD (progressive disease).

## Examples

The present invention is described in more detail below with reference to Examples; however, the present invention is not limited to these Examples, and many modifications can be made by a person who has ordinary knowledge in this field within the technical idea of the present invention.

### Example 1: Study on Administration Schedule of Compound 1 Using Nude Mouse Model Subcutaneously Transplanted with OVCAR-3

Human ovarian cancer cell line OVCAR-3 (American Type Culture Collection (ATCC)) that had been subcutaneously subcultured in mice was cut to about 2 mm squares and subcutaneously transplanted into 5-week-old male BALB/cAJcl-nu/nu mice (CLEA Japan, Inc.) using a transplantation needle.

When the tumor grew to a tumor volume of about 100 to 200 $mm^3$, the animals were divided into groups so that the groups had a uniform tumor volume. The grouping day was defined as Day 1.

The tumor volume (TV) was calculated using the following formula.

$$TV = (\text{major axis} \times \text{minor axis} \times \text{minor axis}) \times 1/2$$

The administration groups were set as shown in the table below. Requisite amounts of Compound 1 were weighed, and each was individually suspended in a 0.5% (w/v) hydroxypropyl methylcellulose (HPMC) aqueous solution to prepare administration liquids having appropriate concentrations. For the control group, a 0.5% (w/v) HPMC aqueous solution was used as an administration liquid.

The administration amount was 10 mL/kg body weight, and each administration liquid was individually injected into the stomach of the mice with a feeding needle.

Table 1

| Group name/ compound name | Administration amount (mg/kg/day) | Administration schedule | Administration route | n number |
|---|---|---|---|---|
| Control (0.5% HPMC)* | - | Administration for 7 consecutive days, Days 1-21 | Oral | 5 |
| Compound 1 | 16 | Administration for 7 consecutive days, Days 1-21 | Oral | 5 |
| Compound 1 | 24 | Administration for 7 consecutive days, Days 1-21 | Oral | 5 |
| Compound 1 | 24 | 4-administration with 3-withdrawal, Days 1-4, 8-11, and 15-18 | Oral | 5 |
| *: 0.5% (w/v) HPMC aqueous solution | | | | |

<u>Antitumor Effect</u>

Twice a week, the major axis and the minor axis of each tumor were measured from the skin with an electronic caliper, and the following items were further calculated. These were used as indexes to confirm the presence or absence of an antitumor effect.

$$\text{Tumor volume (TVn)} = (\text{major axis} \times \text{minor axis} \times \text{minor axis}) \times 1/2$$

$$\text{Relative tumor volume (RTVn)} = \text{TVn}/\text{TV1}$$

$$\text{T/C(\%)} = (\text{average RTVn of administration group})/(\text{average RTVn of control group}) \times 100$$

(n indicates each measurement day.)

Body Weight Change

On the same days as the tumor volume measurement, the body weight (BW) of the mice was measured with an electronic balance, and the body weight change (BWC) was further calculated.

$$\text{BWCn(\%)} = (\text{BWn}-\text{BW1})/\text{BW1} \times 100$$

(n indicates each measurement day.)

Fig. 1 shows the results. As is clear from Fig. 1, an excellent antitumor effect was exhibited without serious weight loss in each of the Compound 1 administration groups in the *in vivo* test. There were no significant differences in antitumor effects and side effects between administration of Compound 1 for 7 consecutive days and 4-administration with 3-withdrawal of Compound 1.

Example 2: Study on Administration Schedule of Compound 1 Used in Combination with Compound 2 Using Nude Mouse Model Subcutaneously Transplanted with Human AN3CA Tumor

Human endometrial cancer-derived cell line AN3CA (American Type Culture Collection (ATCC)) that had been subcutaneously subcultured in mice was cut to about 2 mm squares and transplanted in the vicinity of the last right-side rib of 6-week-old male BALB/cAJcl-nu/nu mice (CLEA Japan, Inc.) using a transplantation needle.

When the tumor grew to a tumor volume of about 50 to 200 $mm^3$, the animals were divided into groups so that the groups had a uniform tumor volume. The grouping day was defined as Day 0.

The tumor volume (TV) was calculated using the following formula.

$$\text{TV} = (\text{major axis} \times \text{minor axis} \times \text{minor axis}) \times 1/2$$

The administration groups were set as shown in the table below. Requisite amounts of Compound 1 and Compound 2 were weighed, and each was individually suspended in a 0.5% (w/v) hydroxypropyl methylcellulose (HPMC) aqueous solution to prepare administration liquids having appropriate concentrations. For the control group, a 0.5% (w/v) HPMC aqueous solution was used as an administration liquid.

Table 2

| Group name/ compound name | Administration amount (mg/kg/day) | Administration schedule | Administration route | n number |
|---|---|---|---|---|
| Control (0.5% HPMC)* | - | Administration for 7 consecutive days, Days 1-14 | Oral | 6 |
| Compound 2 | 15 | Administration for 7 consecutive days, Days 1-14 | Oral | 6 |
| Compound 1 + Compound 2 | 4+15 | Administration for 7 consecutive days, Days 1-14 + administration for 7 consecutive days, Days 1-14 | Oral | 6 |

(continued)

| Group name/ compound name | Administration amount (mg/kg/day) | Administration schedule | Administration route | n number |
|---|---|---|---|---|
| Compound 1 + Compound 2 | 8+15 | Administration for 7 consecutive days, Days 1-14 + administration for 7 consecutive days, Days 1-14 | Oral | 6 |
| Compound 1 + Compound 2 | 8+15 | 4-administration with 3-withdrawal, Days 1-4 and 8-11 + administration for 7 consecutive days, Days 1-14 | Oral | 6 |

Twice a week, the major axis and the minor axis of each tumor were measured from the skin with an electronic caliper, and the following items were further calculated. These were used as indexes to confirm the presence or absence of an antitumor effect.

$$\text{Tumor volume (TVn)} = (\text{major axis} \times \text{minor axis} \times \text{minor axis}) \times 1/2$$

$$\text{Relative tumor volume (RTVn)} = \text{TVn/TV0}$$

$$\text{T/C(\%)} = (\text{average RTVn of administration group})/(\text{average RTVn of control group}) \times 100$$

(n indicates each measurement day.)

Body Weight Change

On the same days as the tumor volume measurement, the body weight (BW) of the mice was measured with an electronic balance, and the body weight change (BWC) was further calculated.

$$\text{BWCn (\%)} = (\text{BWn}-\text{BW1})/\text{BW1} \times 100$$

(n indicates each measurement day.)

Fig. 2 shows the results. As is clear from Fig. 2, an excellent antitumor effect was exhibited without serious weight loss in each of the compound 1 + compound 2 administration groups in the *in vivo* test. There were no significant differences in antitumor effects and side effects between administration of Compound 1 for 7 consecutive days and 4-administration with 3-withdrawal of Compound 1.

Example 3: Study on Administration Schedule of Compound 1 in Humans

This test was performed to determine the recommend dose and administration schedule of Compound 1 and investigate the safety profile of Compound 1 for patients with advanced solid cancers for which standard treatment options are not available.

In this Example, first, the recommend doses for the schedule of administration for 7 consecutive days and the schedule of 4-administration with 3-withdrawal were determined. In the schedule of administration of Compound 1 for 7 consecutive days, Compound 1 was orally administered consecutively once a day for three courses for a total duration of 21 days, with one course taken as 7 days. In 4-administration with 3-withdrawal, Compound 1 was orally administered once a day on day 1 to day 4, day 8 to day 11, and day 15 to day 18 of three courses for a total duration of 21 days, with one course taken as 7 days.

In the schedule of administration for 7 consecutive days, the administration amount taken at one time started at 4 mg/body/day. In the schedule of 4-administration with 3-withdrawal, the administration amount taken at one time started at 32 mg/body/day. The test used a 3+3 design. The dose levels were as follows: level 1 was 4 mg/body/day,

level 2 was 8 mg/body/day, level 3 was 16 mg/body/day, level 4 was 24 mg/body/day, level 5 was 32 mg/body/day, level 6 was 44 mg/body/day, level 7 was 60 mg/body/day, level 8 was 80 mg/body/day, level 9 was 100 mg/body/day, level 10 was 120 mg/body/day, and level 11 was 160 mg/body/day.

In the present invention, the 3+3 design was performed in the following manner. Administration is performed to three or more patients at each dose level. At each dose level, if none of the first three patients experiences a DLT for three courses for a total duration of 21 days, the dose level proceeds to the next higher dose level, which is one level higher. If one of the first three patients experiences a DLT, three additional patients are treated at the same dose level. If none of the three additional patients experiences a DLT at the time of completion of three courses for a total duration of 21 days, the dose level proceeds to the dose level one level higher. If one or more of the three additional patients experience a DLT, the at least six patients are enrolled as patients subjected to DLT evaluation at the dose level one level lower to determine the recommend dose. If two or more of the first three patients experience a DLT, the dose level does not proceed, and the at least six patients are enrolled as patients subjected to DLT evaluation at the dose level one level lower to determine the recommend dose. Whether the level proceeds is determined by the investigator and the sponsor based on comprehensive considerations of not only the information on DLTs, but also all related toxicities regardless of whether they are DLTs, and drugs used in combination/combination therapy from a clinical perspective.

In this Example, the severity of adverse events was graded using CTCAE Ver. 4.03.

Definition of Dose-Limited Toxicity (DLT)

A DLT is a toxicity that is the reason why the dose cannot be further increased when a novel anticancer agent is administered to a patient.

For the evaluation of DLTs, only side effects that occurred during the first three courses for a total duration of 21 days in each administration method were used.

DLTs are specifically defined as follows.

Hematologic Toxicities

a) Grade 4 neutropenia lasting more than 7 days
b) Grade 4 thrombocytopenia or grade 3 thrombocytopenia with bleeding requiring blood transfusion
c) Febrile neutropenia (with an absolute neutrophil count (ANC) of less than 1000/$mm^3$, and a fever of more than 38.3°C (101°F) even once or a sustained fever of 38°C (100.4°F) or more for more than 1 hour

Non-hematologic Toxicities

a) Symptomatic Grade 4 hyperglycemia (fasting blood glucose: more than 500 mg/dL)
b) Grade 3 hyperglycemia (fasting blood glucose: more than 250 mg/dL) that lasts more than 7 days and that cannot be treated by intensive diabetes treatment with insulin, oral diabetes drug, or the like; or asymptomatic Grade 4 hyperglycemia
c) Grade 3 or higher skin toxicity that cannot be treated by aggressive skin treatment with a topical steroid, an oral corticosteroid, or the like
d) Grade 3 or higher nausea/vomiting that lasts more than 48 hours and that cannot be treated by aggressive antiemetic therapy with serotonin (5-hydroxytryptamine-3 (5-$HT_3$)) receptor antagonist (e.g., ondansetron) or the like
e) Grade 3 or higher diarrhea that lasts more than 48 hours and in which an antidiarrheal is not effective
f) Other grade 3 or higher non-hematologic toxicities

Others

a) Grade 2 or higher side effect requiring a prescribed withdrawal period of 20% or more of the scheduled administration period during three courses for a total duration of 21 days
b) Case in which administration cannot be started within 2 weeks from the scheduled administration start day of the fourth course due to a side effect higher than grade 2
c) For aberrations in laboratory data (excluding the above-mentioned hematologic toxicities and non-hematologic

toxicities) and transient signs and symptoms, the investigator and the sponsor determine whether they correspond to DLTs.

The recommend doses were determined based on the incidence of a DLT in each schedule, safety profile, efficacy profile, and the opinion of a committee of physicians who were experts independent of the sponsor and the investigator.

Patients with breast cancer, bladder cancer, colorectal cancer, pancreatic neuroendocrine tumors, gallbladder cancer, anal cancer, and the like were enrolled in this test.

As a result, the maximum tolerable administration amounts for administration for 7 consecutive days and 4-administration with 3-withdrawal were respectively 16 mg/body/day and 24 mg/body/day, which were determined to be the recommend doses.

Example 4: Study on Pharmacokinetics of Compound 1 in Humans

The pharmacokinetics of the recommend doses of Compound 1 in administration for 7 consecutive days and 4-administration with 3-withdrawal were measured in patients with advanced solid cancers for which standard treatment options were not available.

In the administration for 7 consecutive days, Compound 1 was orally administered once a day at a dose of 16 mg/body/day for three courses for a total duration of 21 days, with one course taken as 7 days. Blood samples were taken over time on day 1 and day 21, which was the last administration day of the three courses, to measure the pharmacokinetics.

In the 4-administration with 3-withdrawal, Compound 1 was orally administered once a day at a dose of 24 mg/body/day for three courses for a total duration of 21 days, with one course taken as 7 days. Blood samples were taken over time on day 1 and day 18, which was the last administration day of the three courses, to measure the pharmacokinetics. Blood samples were also taken immediately before administration on day 3, day 4, and day 8.

Fig. 3 shows the blood concentrations of Compound 1 on day 1 (Fig. 3, top) and day 21 or 18 (Fig. 3, bottom), which was the last administration day of the three courses.

The blood half-life of Compound 1 was about 49 hours. A comparison between the blood drug concentrations on day 1 and day 21 in the schedule of administration for 7 consecutive days, and a comparison between the blood drug concentrations on day 1 and day 18 in the schedule of 4-administration with 3-withdrawal revealed that the blood drug concentrations were clearly higher on day 21 in the administration for 7 consecutive days and on day 18 in the 4-administration with 3-withdrawal than those on day 1. In the 4-administration with 3-withdrawal of Compound 1, the blood concentrations of the drug remaining before administration on day 3 and day 4 were 25.7 ng/mL and 35.7 ng/mL, respectively. The blood drug concentrations on day 21 in the administration for 7 consecutive days and on day 18 in the 4-administration with 3-withdrawal showed that Compound 1 has accumulation properties.

Example 5: Study on Optimal Dosage Regimen of Compound 1 in Humans

With regard to Example 3, the optimal dosage regimen of Compound 1 was studied. The safety profiles and efficacy profiles when the recommend doses were administered in the schedule of administration for 7 consecutive days and the schedule of 4-administration with 3-withdrawal were compared in patients with advanced solid cancers for which standard treatment options were not available.

For the safety profiles, non-prescribed withdrawal periods were compared. The non-prescribed withdrawal period is a comprehensive index of safety profiles.

In this Example, dose modification criteria in the case of the occurrence of a toxicity for which a causal relationship to the drug cannot be denied were shown. Dose modification criteria for non-hematologic toxicities, dose modification criteria for hematologic toxicities, and dose modification criteria for other toxicities were set. When any of these criteria was met, a prescribed day to perform drug administration was changed to a day on which the drug is not administered, i.e., a non-prescribed withdrawal period.

Dose Modification Criteria for Non-hematologic Toxicities

The dose modification criteria for non-hematologic toxicities for which a causal relationship to the drug cannot be denied were set as follows.

With regard to fasting blood glucose, if symptomatic grade 4 hyperglycemia or grade 3 or higher hyperglycemia for more than 7 days develops, the administration is discontinued (non-prescribed withdrawal). If the condition recovers to grade 1 or grade 0, the administration can be resumed. When the administration is resumed, the administration amount is reduced by one level from the original administration amount. However, for adverse events (e.g., fatigue and skin

dryness) that are judged to be less likely to become serious or worsen to life-threatening events, the administration of the drug can be continued at the same administration amount without dose reduction or administration discontinuation, regardless of the grades of the events.

In the case of DLTs or grade 3 or higher non-hematologic toxicities as other toxicities, the administration is discontinued (non-prescribed withdrawal). If the condition recovers to grade 1 or less or to the baseline, the administration can be resumed. As grade 3 or higher non-hematologic toxicities, grade 3 nausea and/or vomiting that could be treated by intensive antiemetic therapy, grade 3 diarrhea in which an antidiarrheal was effective, and grade 3 skin toxicity that could be treated by intensive skin treatment with, for example, a topical steroid and/or an oral corticosteroid are excluded. When the administration is resumed, the administration amount is reduced by one level from the original administration amount. However, for adverse events (e.g., fatigue and skin dryness) that are judged to be less likely to become serious or worsen to life-threatening events, the administration of the drug can be continued at the same administration amount without dose reduction or administration discontinuation, regardless of the grades of the events.

Dose Modification Criteria for Hematologic Toxicities

The dose modification criteria for hematologic toxicities for which a causal relationship to the drug cannot be denied were set as follows.

If the neutrophil count is less than 500/$mm^3$, the administration is discontinued (non-prescribed withdrawal). If the neutrophil count recovers to 1500/$mm^3$ or more, the administration can be resumed. When the administration is resumed, the administration amount does not need to be changed in the case of asymptomatic neutropenia lasting 7 days or less. In the case of asymptomatic neutropenia lasting more than 7 days or neutropenia with a symptom (neutropenia with an infectious disease or fever), the administration amount is reduced by one level from the original administration amount.

If the platelet count is less than 25000/$mm^3$, the administration is discontinued (non-prescribed withdrawal). If the platelet count recovers to 75000/$mm^3$ or more, the administration can be resumed. When the administration is resumed, the administration amount is reduced by one level from the original administration amount.

Dose Modification Criteria for Other Toxicities

The dose modification criteria for other toxicities for which a causal relationship to the drug cannot be denied and to which none of the above cases applies were set as follows.

With regard to other toxicities deemed by the investigator to require the discontinuation of administration, the administration is discontinued (non-prescribed withdrawal). If the condition recovers to grade 1 or less or to the baseline, the administration can be resumed. When the administration is resumed, the administration amount does not need to be changed.

Table 3 shows the results of the safety profiles. A comparison between the two administration groups of the recommend doses revealed the following: in the administration for 7 consecutive days, three of six patients required non-prescribed withdrawal due to side effects, and the average non-prescribed withdrawal period was 3.7 days; whereas in the 4-administration with 3-withdrawal, one of six patients required non-prescribed withdrawal, and the non-prescribed withdrawal period was 2 days. These results showed that with regard to Compound 1, the 4-administration with 3-withdrawal is superior to the administration for 7 consecutive days in terms of the control of side effects.

Table 3

| Administration schedule | Administration for 7 consecutive days (N=6) | 4-administration with 3-withdrawal (N=6) |
|---|---|---|
| Dose | 16 mg/body/day | 24 mg/body/day |
| Total administration amount/ courses | 336 mg/three courses | 288 mg/three courses |
| Non-prescribed withdrawal | 3/6 patients | 1/6 patients |
| Non-prescribed withdrawal period | 3.7 days | 2.0 days |

The efficacy profiles of the recommend doses for the individual schedules were compared by drawing waterfall plots of an antitumor effect in each patient (Fig. 4). Further, the results of the antitumor effect were compared by RECIST evaluation.

As shown in the waterfall plots in Fig. 4, the results revealed that the 4-administration with 3-withdrawal is

superior to the administration for 7 consecutive days in terms of a tumor shrinkage effect. Moreover, the results of RECIST evaluation revealed that two of the six patients had stable disease (SD) in the 4-administration with 3-withdrawal, whereas all of the patients had progressive disease (PD) in the administration for 7 consecutive days. As is clear from these results, with regard to Compound 1, the 4-administration with 3-withdrawal is superior to the administration for 7 consecutive days in terms of an antitumor effect.

From the above, it was clarified that administration of Compound 1 based on 4-administration with 3-withdrawal is superior in terms of obtaining an excellent antitumor effect and controlling side effects in human tumor patients. This is a surprising result that is unexpected from animal experiments in mice and findings about other compounds.

Example 6: Phase I/II Clinical Trial of Combination of 4-administration with 3-withdrawal of Compound 1 and Administration of Compound 2 for 7 Consecutive Days

The purpose of this test is to examine the safety and tolerability of a combination of 4-administration with 3-withdrawal of Compound 1 and administration of Compound 2 for 7 consecutive days and determine the recommend doses in human patients with solid cancers. This is a phase I/II, open-label, non-randomized, dose-escalation, multicenter trial to evaluate the safety, tolerability, efficacy, PK, pharmacodynamics, and predictive biomarkers of the combined use of Compound 1 and Compound 2 in patients with advanced or metastatic solid cancers for which standard treatment options are not available. The dosage regimen of Compound 2 is fixed, and the dose of Compound 1 is escalated using a 3+3 design to determine the recommended dosage regimen. The efficacy and safety are then examined using the determined dosage regimen.

In this test, the administration is performed for three courses for a total duration of 21 days, with one course taken as 7 days. Specifically, Compound 1 is orally administered once a day in the range of 4 mg to 160 mg/body/day for 21 consecutive days, starting at 8 mg/body/day, according to the 3+3 design. Compound 2 is administered once a day in the range of 12 mg to 20 mg for 21 consecutive days, starting at 20 mg/body/day. The course is repeated until the patient meets the set discontinuation criteria. When 4-administration with 3-withdrawal or administration once a week is considered to be desirable as the dosage regimen of Compound 1, based on the profiles of the safety, tolerability, efficacy, pharmacokinetics, pharmacodynamic biomarkers, etc., the dosage regimen of Compound 1 can be changed.

When the administration method for Compound 1 is changed from administration for 7 consecutive days to 4-administration with 3-withdrawal, Compound 1 is orally administered once a day in the range of 4 mg to 160 mg/body/day based on 4-administration with 3-withdrawal for three courses for a total duration of 21 days, with one course taken as 7 days, according to the 3+3 design. Compound 2 is administered once a day in the range of 12 mg to 20 mg/body/day for 21 consecutive days.

In the schedule of administration for 7 consecutive days, the administration amount taken at one time starts at 8 mg/body/day. In the schedule of 4-administration with 3-withdrawal, the administration starts at the administration amount judged to be preferable based on the results of the administration for 7 days. The test uses the 3+3 design. The dose levels are as follows: level -1 is 4 mg/body/day, level 1 is 8 mg/body/day, level 2 is 12 mg/body/day, level 3 is 16 mg/body/day, level 4 is 20 mg/body/day, level 5 is 24 mg/body/day, level 6 is 32 mg/body/day, and level 7 is 44 mg/body/day. Based on the profiles of safety, tolerability, efficacy, pharmacokinetics, pharmacodynamic biomarkers, etc., the administration amount can be further increased. In this case, the administration amount is, for example, 60 mg/body/day, 80 mg/body/day, 100 mg/body/day, 12 mg/body/day, or 160 mg/body/day.

The administration of Compound 2 or a salt thereof starts from administration at 20 mg/body/day once a day for 21 consecutive days. The administration amount of Compound 2 or a salt thereof can be reduced depending on the occurrence of adverse events. The administration amount of Compound 2 or a salt thereof can be reduced to 16 mg/body/day or 12 mg/body/day.

In the present invention, the 3+3 design is performed in the following manner. Administration is performed to three or more patients at each dose level. At each dose level, if none of the first three patients experiences a DLT for three courses for a total duration of 21 days, the dose level proceeds to the next higher dose level, which is one level higher. If one of the first three patients experiences a DLT, three additional patients are treated at the same dose level. If none of the three additional patients experiences a DLT at the time of completion of three courses for a total duration of 21 days, the dose level proceeds to the dose level one level higher. If one or more of the three additional patients experience a DLT, the at least six patients are enrolled as patients subjected to DLT evaluation at the dose level one level lower to determine the recommend dose. If two or more of the first three patients experience a DLT, the dose level does not proceed, and the at least six patients are enrolled as patients subjected to DLT evaluation at the dose level one level lower to determine the recommend dose. Whether the level proceeds is determined by the investigator and the sponsor based on comprehensive considerations of not only the information on DLTs, but also all related toxicities regardless of whether they are DLTs, and drugs used in combination/combination therapy from a clinical perspective.

Definition of Dose-Limited Toxicity (DLT)

A DLT is a toxicity that is the reason why the dose cannot be further increased when a novel anticancer agent is administered to a patient.
For the evaluation of DLTs, only side effects that occurred during the first three courses for a total duration of 21 days of each administration method are used.
DLTs are specifically defined as follows.

Hematologic Toxicities

1) Grade 4 neutropenia lasting more than 7 days
2) Grade 4 thrombocytopenia or grade 3 thrombocytopenia with bleeding requiring blood transfusion
3) Febrile neutropenia (with an absolute neutrophil count (ANC) of less than 1000/$mm^3$, and a fever of more than 38.3°C (101°F) even once or a sustained fever of 38°C (100.4°F) or more for more than 1 hour

Non-hematologic Toxicities

1) Grade 4 hyperglycemia
2) Grade 3 hyperglycemia that lasts more than 7 days and that cannot be treated by intensive diabetes treatment with insulin or the like
3) Grade 3 or higher skin toxicity that cannot be treated by aggressive skin treatment with a topical steroid, an oral corticosteroid, or the like
4) Hyperphosphatemia with an inorganic phosphate (Pi) value of >10 mg/dL and/or a Pi value of >7 mg/dL for 7 days or more although a treatment to lower the Pi value is performed for 7 days
5) Grade 1 or higher corneal disorder associated with calcification
6) Increase in creatinine, Pi, calcium Creatinine > 1.5 × upper limit of normal (ULN) continues for 7 days or more, and Pi>5.5 mg/dL although a treatment to lower Pi is performed for 7 days and/or corrected calcium × Pi>55 mg/dL although a treatment to lower Pi is performed for 7 days
7) Grade 2 hypercalcemia lasting more than 7 days or grade 3 or higher hypercalcemia
8) New ectopic calcification in soft tissue judged by the investigator
9) Grade 3 or higher nausea/vomiting that lasts more than 48 hours and that cannot be treated by aggressive antiemetic therapy with serotonin (5-hydroxytryptamine-3 (5-$HT_3$)) receptor antagonist (e.g., ondansetron) or the like

    e) Grade 3 or higher diarrhea that lasts more than 48 hours and in which an antidiarrheal is not effective
    f) Other grade 3 or higher non-hematologic toxicities

Others

a) Grade 2 or higher side effect that prevents completion of three courses for a total duration of 21 days
b) Case in which administration cannot be started within 2 weeks from the scheduled administration start day of the fourth course due to a side effect higher than grade 2

The recommend doses are determined based on the incidence of a DLT in each schedule, safety profile, efficacy profile, and the opinion of a committee of physicians who are experts independent of the sponsor and the investigator.
In this test, the severity of adverse events is graded using CTCAE Ver. 5.0.
The combination of 4-administration with 3-withdrawal of Compound 1 or a salt thereof and administration of Compound 2 or a salt thereof for 21 consecutive days can provide antitumor effects that cannot be expected from monotherapies separately using each of them. For example, in an embodiment of the present invention, it can be found that this combined use has a higher degree of effects than at least one of the measurement results of treatment with either of them. Moreover, for example, in an embodiment of the present invention, a synergistic effect can be obtained by the combination of 4-administration with 3-withdrawal of Compound 1 or a salt thereof and administration of Compound 2 or a salt thereof for 21 consecutive days.

The antitumor effects are evaluated according to the RECIST guidelines, version 1.1. The combination of 4-administration with 3-withdrawal of Compound 1 or a salt thereof and administration of Compound 2 or a salt thereof for 7 consecutive days can be more effective than the single use of either of them, according to at least one of the following measurement results: objective response rate (ORR), disease control rate (DCR), duration of response (DOR), progression-free survival (PFS), overall survival (OS), and time to response (TTR).

## Claims

1. A therapeutic agent for treating a malignant tumor in a human patient, comprising trans-3-amino-1-methyl-3-(4-(3-phenyl-5H-imidazo[1,2-c]pyrido[3,4-e][1,3]oxazin-2-yl)phenyl)cyclobutanol or a salt thereof,
wherein
the agent is for use in administration to a patient in need of the treatment according to an administration schedule, the administration schedule including one course for a duration of 7 days in which the trans-3-amino-1-methyl-3-(4-(3-phenyl-5H-imidazo[1,2-c]pyrido[3,4-e][1,3]oxazin-2-yl)phenyl)cyclobutanol or a salt thereof is administered in a dose of 4 mg/body/day to 160 mg/body/day in terms of trans-3-amino-1-methyl-3-(4-(3-phenyl-5H-imidazo[1,2-c]pyrido[3,4-e][1,3]oxazin-2-yl)phenyl)cyclobutanol for 4 consecutive days, followed by a prescribed withdrawal period of 3 days.

2. The therapeutic agent according to claim 1, wherein the trans-3-amino-1-methyl-3-(4-(3-phenyl-5H-imidazo[1,2-c]pyrido[3,4-e][1,3]oxazin-2-yl)phenyl)cyclobutanol or a salt thereof is administered once a day on an administration day.

3. The therapeutic agent according to claim 1 or 2, wherein the administration schedule includes three courses for a total duration of 21 days, with one course taken as 7 days, in which the trans-3-amino-1-methyl-3-(4-(3-phenyl-5H-imidazo[1,2-c]pyrido[3,4-e][1,3]oxazin-2-yl)phenyl)cyclobutanol or a salt thereof is administered for 4 consecutive days each from day 1 to day 4, day 8 to day 11, and day 15 to day 18, and prescribed withdrawal is performed from day 5 to day 7, day 12 to day 14, and day 19 to day 21.

4. The therapeutic agent according to any one of claims 1 to 3, wherein the dose of the trans-3-amino-1-methyl-3-(4-(3-phenyl-5H-imidazo[1,2-c]pyrido[3,4-e][1,3]oxazin-2-yl)phenyl)cyclobutanol or a salt thereof is 4 mg/body/day to 44 mg/body/day.

5. The therapeutic agent according to any one of claims 1 to 4, wherein the dose of the trans-3-amino-1-methyl-3-(4-(3-phenyl-5H-imidazo[1,2-c]pyrido[3,4-e][1,3]oxazin-2-yl)phenyl)cyclobutanol or a salt thereof is 16 mg/body/day to 32 mg/body/day.

6. The therapeutic agent according to any one of claims 1 to 5, wherein the dose of the trans-3-amino-1-methyl-3-(4-(3-phenyl-5H-imidazo[1,2-c]pyrido[3,4-e][1,3]oxazin-2-yl)phenyl)cyclobutanol or a salt thereof is 24 mg/body/day.

7. The therapeutic agent according to any one of claims 1 to 6, wherein the malignant tumor is lung cancer, breast cancer, renal cancer, sarcoma, hematopoietic tumor, central nervous system tumor, liver cancer, ovarian cancer, gastric cancer, rhabdomyosarcoma, endometrial cancer, urinary tract cancer, prostate cancer, colorectal cancer, pancreatic cancer, vulvar cancer, thyroid cancer, skin cancer, or biliary tract cancer.

8. The therapeutic agent according to any one of claims 1 to 7, wherein the malignant tumor is lung cancer, endometrial cancer, urinary tract cancer, breast cancer, prostate cancer, colorectal cancer, biliary tract cancer, ovarian cancer, or multiple myeloma.

9. The therapeutic agent according to any one of claims 1 to 8, wherein the malignant tumor is lung cancer, breast cancer, bladder cancer, colon cancer, rectal cancer, gallbladder cancer, or anal cancer.

10. The therapeutic agent according to any one of claims 1 to 9, wherein a side effect caused by the trans-3-amino-1-methyl-3-(4-(3-phenyl-5H-imidazo[1,2-c]pyrido[3,4-e][1,3]oxazin-2-yl)phenyl)cyclobutanol or a salt thereof is reduced.

11. The therapeutic agent according to any one of claims 1 to 10, wherein a side effect of maculopapular rash caused by the trans-3-amino-1-methyl-3-(4-(3-phenyl-5H-imidazo[1,2-c]pyrido[3,4-e][1,3]oxazin-2-yl)phenyl)cyclobutanol

or a salt thereof is reduced.

**12.** The therapeutic agent according to any one of claims 1 to 11, wherein a side effect of hyperglycemia caused by the trans-3-amino-1-methyl-3-(4-(3-phenyl-5H-imidazo[1,2-c]pyrido[3,4-e][1,3]oxazin-2-yl)phenyl)cyclobutanol or a salt thereof is reduced.

**13.** The therapeutic agent according to any one of claims 1 to 12, which is for a combined use of the trans-3-amino-1-methyl-3-(4-(3-phenyl-5H-imidazo[1,2-c]pyrido[3,4-e][1,3]oxazin-2-yl)phenyl)cyclobutanol or a salt thereof with (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof,
wherein the (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered according to the administration schedule, the administration schedule including administration of the (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof in combination for 7 consecutive days.

**14.** The therapeutic agent according to claim 13, wherein the (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one or a salt thereof is administered in combination in a dose of 12 mg/body/day to 20 mg/body/day in terms of (S)-1-(3-(4-amino-3-((3,5-dimethoxyphenyl)ethynyl)-1H-pyrazolo[3,4-d]pyrimidin-1-yl)pyrrolidin-1-yl)prop-2-en-1-one for 7 consecutive days.

**15.** A method for treating a malignant tumor in a human patient,
the method comprising administering trans-3-amino-1-methyl-3-(4-(3-phenyl-5H-imidazo[1,2-c]pyrido[3,4-e] [1,3] oxazin-2-yl)phenyl)cyclobutanol or a salt thereof to a patient in need of the treatment according to an administration schedule, the administration schedule including one course for a duration of 7 days in which the trans-3-amino-1-methyl-3-(4-(3-phenyl-5H-imidazo[1,2-c]pyrido[3,4-e] [1,3]oxazin-2-yl)phenyl)cyclobutanol or a salt thereof is administered in a dose of 4 mg/body/day to 160 mg/body/day in terms of trans-3-amino-1-methyl-3-(4-(3-phenyl-5H-imidazo[1,2-c]pyrido[3,4-e] [1,3]oxazin-2-yl)phenyl)cyclobutanol for 4 consecutive days, followed by a prescribed withdrawal period of 3 days.

**16.** A pharmaceutical composition for treating a malignant tumor in a human patient, comprising trans-3-amino-1-methyl-3-(4-(3-phenyl-5H-imidazo[1,2-c]pyrido[3,4-e][1,3]oxazin-2-yl)phenyl)cyclobutanol or a salt thereof,
wherein
the trans-3-amino-1-methyl-3-(4-(3-phenyl-5H-imidazo[1,2-c]pyrido[3,4-e][1,3]oxazin-2-yl)phenyl)cyclobutanol or a salt thereof is for use in administration to a patient in need of the treatment according to an administration schedule, the administration schedule including one course for a duration of 7 days in which the trans-3-amino-1-methyl-3-(4-(3-phenyl-5H-imidazo[1,2-c]pyrido[3,4-e][1,3]oxazin-2-yl)phenyl)cyclobutanol or a salt thereof is administered in a dose of 4 mg/body/day to 160 mg/body/day in terms of trans-3-amino-1-methyl-3-(4-(3-phenyl-5H-imidazo[1,2-c]pyrido[3,4-e][1,3]oxazin-2-yl)phenyl)cyclobutanol for 4 consecutive days, followed by a prescribed withdrawal period of 3 days.

**17.** Use of trans-3-amino-1-methyl-3-(4-(3-phenyl-5H-imidazo[1,2-c]pyrido[3,4-e][1,3]oxazin-2-yl)phenyl)cyclobutanol or a salt thereof in the manufacture of a therapeutic agent for a malignant tumor in a human patient,
wherein
the trans-3-amino-1-methyl-3-(4-(3-phenyl-5H-imidazo[1,2-c]pyrido[3,4-e][1,3]oxazin-2-yl)phenyl)cyclobutanol or a salt thereof is for use in administration to a patient in need of the treatment according to an administration schedule, the administration schedule including one course for a duration of 7 days in which the trans-3-amino-1-methyl-3-(4-(3-phenyl-5H-imidazo[1,2-c]pyrido[3,4-e][1,3]oxazin-2-yl)phenyl)cyclobutanol or a salt thereof is administered in a dose of 4 mg/body/day to 160 mg/body/day in terms of trans-3-amino-1-methyl-3-(4-(3-phenyl-5H-imidazo[1,2-c]pyrido[3,4-e][1,3]oxazin-2-yl)phenyl)cyclobutanol for 4 consecutive days, followed by a prescribed withdrawal period of 3 days.

**18.** Use of trans-3-amino-1-methyl-3-(4-(3-phenyl-5H-imidazo[1,2-c]pyrido[3,4-e][1,3]oxazin-2-yl)phenyl)cyclobutanol or a salt thereof for the treatment of a malignant tumor in a human patient,
wherein
the trans-3-amino-1-methyl-3-(4-(3-phenyl-5H-imidazo[1,2-c]pyrido[3,4-e][1,3]oxazin-2-yl)phenyl)cyclobutanol or a salt thereof is for use in administration to a patient in need of the treatment according to an administration schedule, the administration schedule including one course for a duration of 7 days in which the trans-3-amino-1-methyl-3-(4-(3-phenyl-5H-imidazo[1,2-c]pyrido[3,4-e][1,3]oxazin-2-yl)phenyl)cyclobutanol or a salt thereof is administered

in a dose of 4 mg/body/day to 160 mg/body/day in terms of trans-3-amino-1-methyl-3-(4-(3-phenyl-5H-imidazo[1,2-c]pyrido[3,4-e][1,3]oxazin-2-yl)phenyl)cyclobutanol for 4 consecutive days, followed by a prescribed withdrawal period of 3 days.

**19.** Trans-3-amino-1-methyl-3-(4-(3-phenyl-5H-imidazo[1,2-c]pyrido[3,4-e][1,3]oxazin-2-yl)phenyl)cyclobutanol or a salt thereof for use in the treatment of a malignant tumor in a human patient,
wherein
the trans-3-amino-1-methyl-3-(4-(3-phenyl-5H-imidazo[1,2-c]pyrido[3,4-e][1,3]oxazin-2-yl)phenyl)cyclobutanol or a salt thereof is for use in administration to a patient in need of the treatment according to an administration schedule, the administration schedule including one course for a duration of 7 days in which the trans-3-amino-1-methyl-3-(4-(3-phenyl-5H-imidazo[1,2-c]pyrido[3,4-e][1,3]oxazin-2-yl)phenyl)cyclobutanol or a salt thereof is administered in a dose of 4 mg/body/day to 160 mg/body/day in terms of trans-3-amino-1-methyl-3-(4-(3-phenyl-5H-imidazo[1,2-c]pyrido[3,4-e][1,3]oxazin-2-yl)phenyl)cyclobutanol for 4 consecutive days, followed by a prescribed withdrawal period of 3 days.

**20.** A kit for treating a malignant tumor in a human patient, comprising trans-3-amino-1-methyl-3-(4-(3-phenyl-5H-imidazo[1,2-c]pyrido[3,4-e][1,3]oxazin-2-yl)phenyl)cyclobutanol or a salt thereof,
wherein
the trans-3-amino-1-methyl-3-(4-(3-phenyl-5H-imidazo[1,2-c]pyrido[3,4-e][1,3]oxazin-2-yl)phenyl)cyclobutanol or a salt thereof is for use in administration to a patient in need of the treatment according to an administration schedule, the administration schedule including one course for a duration of 7 days in which the trans-3-amino-1-methyl-3-(4-(3-phenyl-5H-imidazo[1,2-c]pyrido[3,4-e][1,3]oxazin-2-yl)phenyl)cyclobutanol or a salt thereof is administered in a dose of 4 mg/body/day to 160 mg/body/day in terms of trans-3-amino-1-methyl-3-(4-(3-phenyl-5H-imidazo[1,2-c]pyrido[3,4-e][1,3]oxazin-2-yl)phenyl)cyclobutanol for 4 consecutive days, followed by a prescribed withdrawal period of 3 days.

Fig. 1

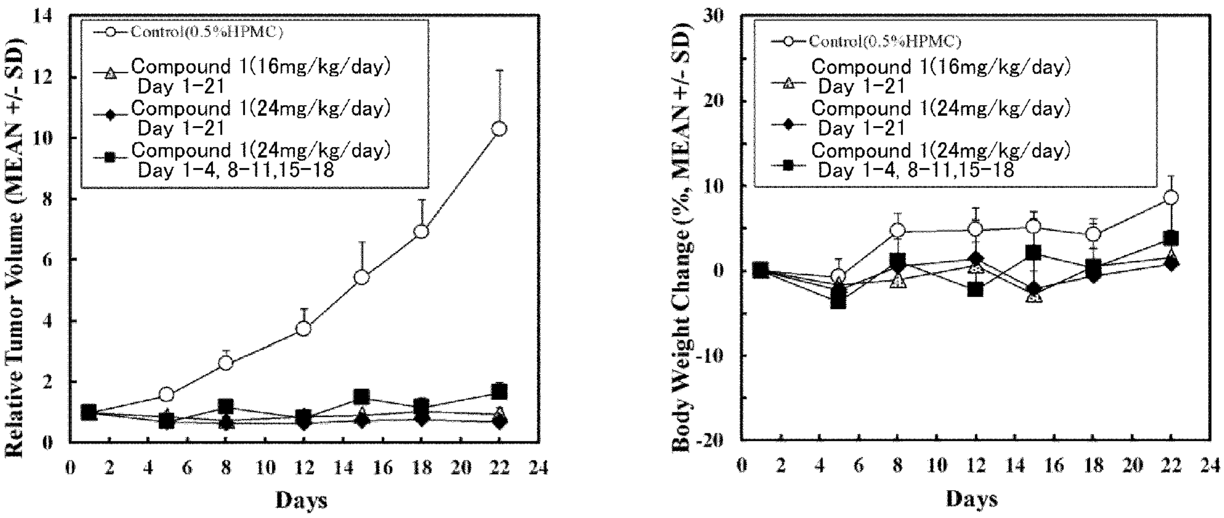
Relative Tumor Volume (MEAN +/- SD)
Days
Control(0.5%HPMC)
Compound 1(16mg/kg/day) Day 1-21
Compound 1(24mg/kg/day) Day 1-21
Compound 1(24mg/kg/day) Day 1-4, 8-11,15-18
Body Weight Change (%, MEAN +/- SD)
Days

Fig. 2

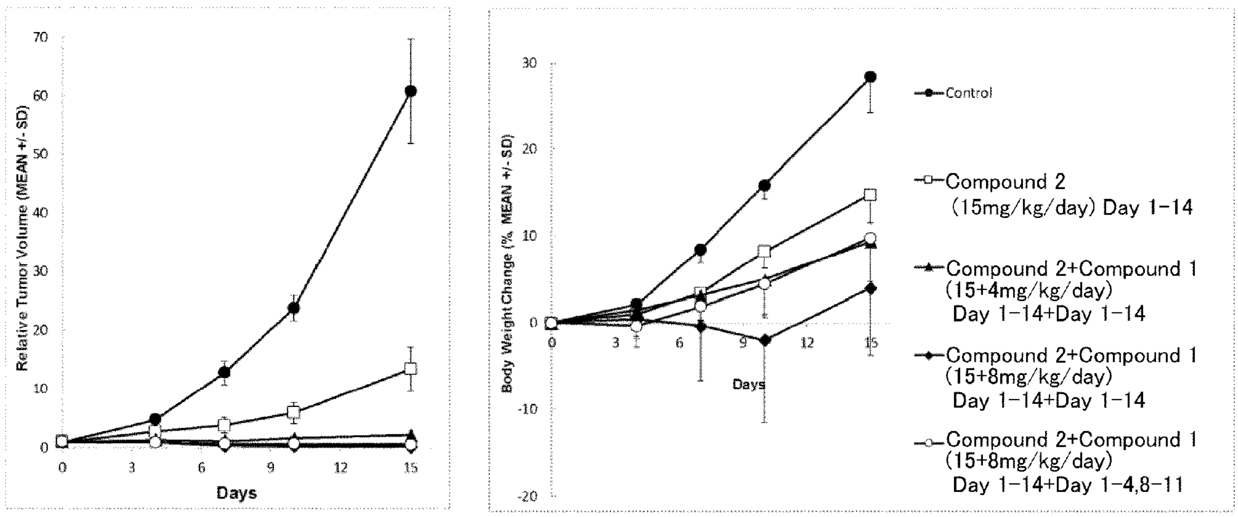
Relative Tumor Volume (MEAN +/- SD)
Days
Body Weight Change (% MEAN +/- SD)
Days
Control
Compound 2 (15mg/kg/day) Day 1-14
Compound 2+Compound 1 (15+4mg/kg/day) Day 1-14+Day 1-14
Compound 2+Compound 1 (15+8mg/kg/day) Day 1-14+Day 1-14
Compound 2+Compound 1 (15+8mg/kg/day) Day 1-14+Day 1-4,8-11

Fig. 3

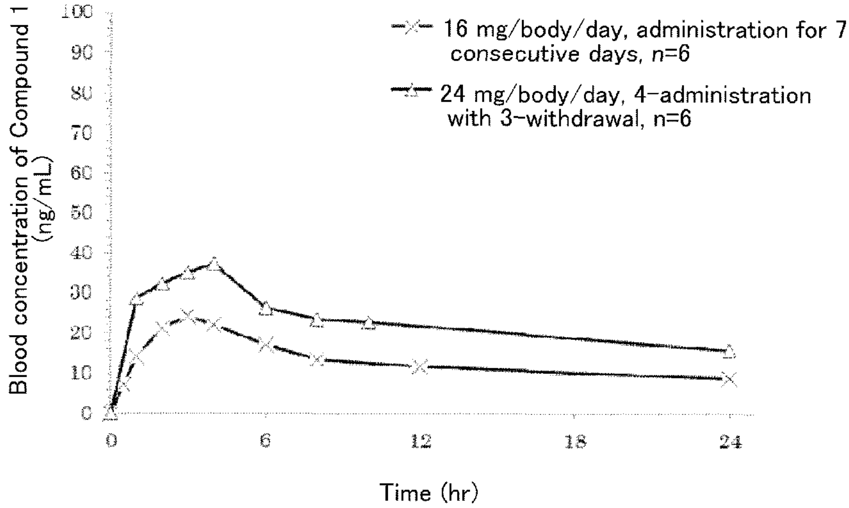
Blood concentration of Compound 1 (ng/mL)
100
90
80
70
60
50
40
30
20
10
0
0
6
12
18
24
Time (hr)
16 mg/body/day, administration for 7 consecutive days, n=6
24 mg/body/day, 4-administration with 3-withdrawal, n=6

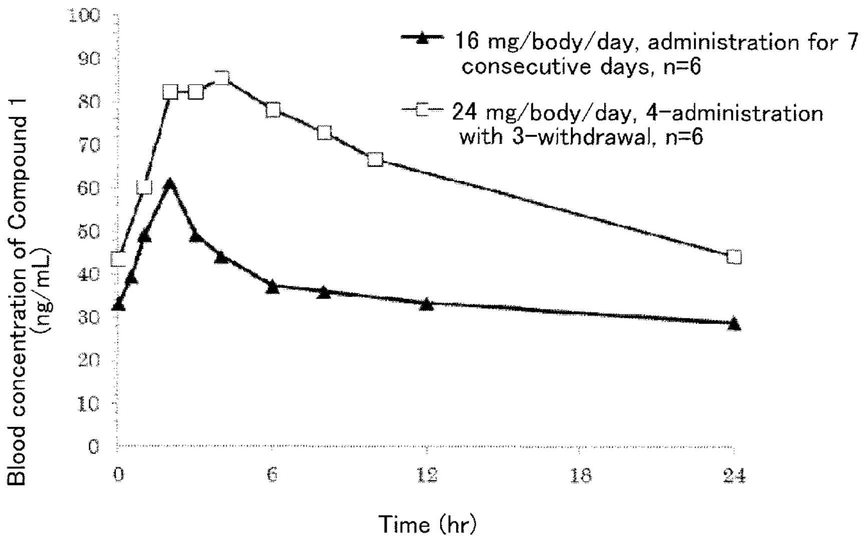
Blood concentration of Compound 1 (ng/mL)
100
90
80
70
60
50
40
30
20
10
0
0
6
12
18
24
Time (hr)
16 mg/body/day, administration for 7 consecutive days, n=6
24 mg/body/day, 4-administration with 3-withdrawal, n=6

Fig. 4

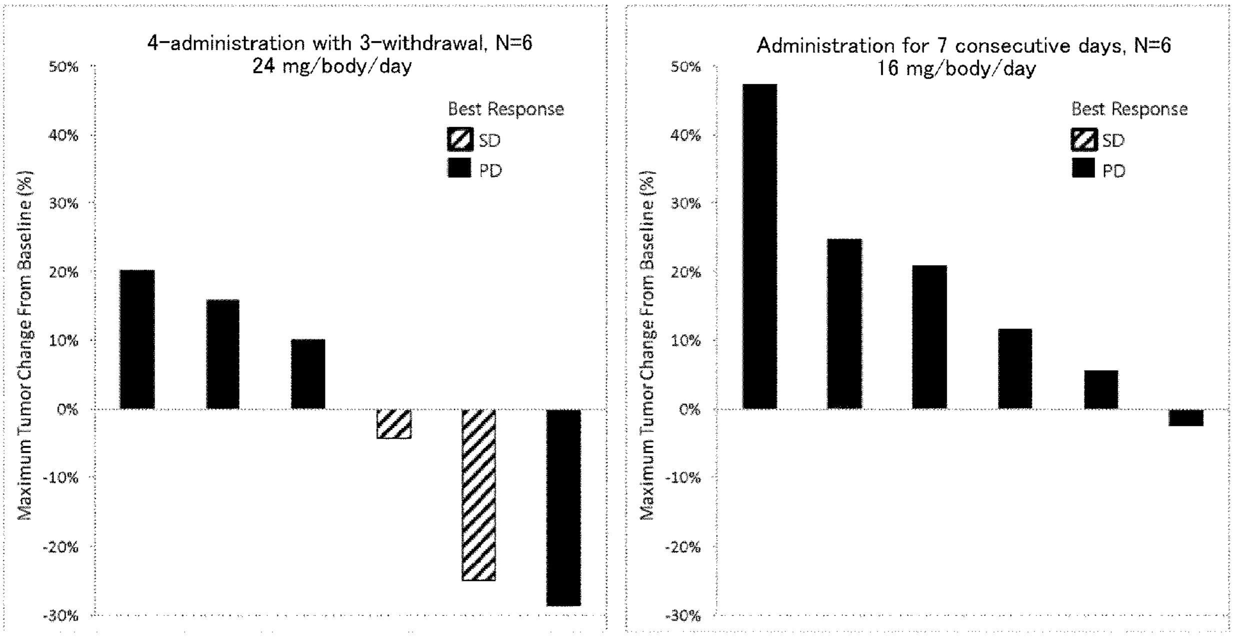
4-administration with 3-withdrawal, N=6
24 mg/body/day
Best Response
SD
PD
Maximum Tumor Change From Baseline (%)
50%
40%
30%
20%
10%
0%
-10%
-20%
-30%
Administration for 7 consecutive days, N=6
16 mg/body/day
Best Response
SD
PD
Maximum Tumor Change From Baseline (%)
50%
40%
30%
20%
10%
0%
-10%
-20%
-30%

**INTERNATIONAL SEARCH REPORT**

International application No. PCT/JP2020/024113

A. CLASSIFICATION OF SUBJECT MATTER
A61P 35/00(2006.01)i; A61P 43/00(2006.01)i; A61K 31/519(2006.01)i; A61K 31/5365(2006.01)i
FI: A61K31/5365; A61P35/00; A61P43/00 121; A61K31/519; A61P43/00 111
According to International Patent Classification (IPC) or to both national classification and IPC

B. FIELDS SEARCHED

Minimum documentation searched (classification system followed by classification symbols)
A61P35/00; A61P43/00; A61K31/519; A61K31/5365

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2014/007217 A1 (TAIHO PHARMACEUTICAL CO., LTD.) 09.01.2014 (2014-01-09) example 32<br>paragraphs [0100], [0102], [0128], [0129], test examples 1-13 | 19<br>1-20 |
| Y | BROWN, Jessica S. et al., "Maximising the potential of AKT inhibitors as anti-cancer treatments", Pharmacology & Therapeutics, 2017, vol. 172, pp. 101-115, ISSN:0163-7258, in particular, page 106, right column, lines 32-65 | 1-20 |
| Y | WO 2017/150725 A1 (TAIHO PHARMACEUTICAL CO., LTD.) 08.09.2017 (2017-09-08) paragraphs [0097], [0098], [0114], [0115] | 13-14 |
| P, X | YUNOKAWA, M. et al., "First-in-human phase I study of TAS-117, an allosteric AKT inhibitor, in patients with advanced sol id tumours", Annals of Oncology, 01 October 2019, vol. 30, supplement 5, page v169, doi: 10.1093/annonc/mdz244.018, ISSN:0923-7534, 456P | 1-12, 15-20 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August 2020 (07.08.2020) | 18 August 2020 (18.08.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/024113

C (Continuation). DOCUMENTS CONSIDERED TO BE RELEVANT

| Category* | Citation of document, with indication, where appropriate, of the relevant passages | Relevant to claim No. |
|---|---|---|
| A | WO 2014/203959 A1 (TAIHO PHARMACEUTICAL CO., LTD.) 24.12.2014 (2014-12-24) whole document | 1-20 |
| A | WO 2012/137870 A1 (TAIHO PHARMACEUTICAL CO., LTD.) 11.10.2012 (2012-10-11) whole document | 1-20 |
| A | WO 2016/136928 A1 (TAIHO PHARMACEUTICAL CO., LTD.) 01.09.2016 (2016-09-01) whole document | 1-20 |

Form PCT/ISA/210 (continuation of second sheet) (January 2015)

**INTERNATIONAL SEARCH REPORT**
Information on patent family members

International application no.
PCT/JP2020/024113

| Patent Documents referred in the Report | Publication Date | Patent Family | Publication Date |
|---|---|---|---|
| WO 2014/007217 A1 | 09 Jan. 2014 | US 2015/0164909 A1 example 32, paragraphs [0172], [0174], [0200]-[0201], test examples 1-12<br>EP 2868660 A1<br>KR 10-2015-0023870 A | |
| WO 2017/150725 A1 | 08 Sep. 2017 | US 2019/0350932 A1 paragraphs [0346], [0373]-[0374]<br>EP 3424505 A1<br>KR 10-2018-0118719 A | |
| WO 2014/203959 A1 | 24 Dec. 2014 | US 2016/0102366 A1 whole document<br>EP 3012327 A1<br>KR 10-2018-0069141 A | |
| WO 2012/137870 A1 | 11 Oct. 2012 | US 2014/0005185 A1 whole document<br>EP 2698372 A1<br>CN 103459400 A<br>KR 10-2014-0039186 A | |
| WO 2016/136928 A1 | 01 Sep. 2016 | US 2018/0030067 A1 whole document<br>EP 3263573 A1<br>CN 107428779 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019115496 A
- WO 2012137870 A
- WO 2013108809 A
- US 20190350932


### Non-patent literature cited in the description


- *Cell,* 2007, vol. 129, 1261-1274
- *Ann Oncol,* 2010, vol. 21, 683-691
- *J Clin Oncol.,* 2011, vol. 29, 4688-4695
- *Cancer Res.,* 2013, vol. 73 (8
- *Clin Cancer Res,* 01 May 2018, vol. 24 (9), 2050-2059
- Characterization of TAS-117, a novel, highly potent and selective inhibitor of AKT. *Poster presentation material for the conference "EORTC-NCI-AACR 2017*
- *European Journal of Cancer,* 2009, vol. 45, 228-247